# EUROPÄISCHE PATENTSCHRIFT

(11) **EP 1 119 334 B1**
(45) Veröffentlichungstag und Bekanntmachung des Hinweises auf die Patenterteilung: **29.01.2003**
(21) Anmeldenummer: 99950688.4
(22) Anmeldetag: 09.10.1999
(51) Int. Cl.: A61J 1/00

(54) **ZWEIKAMMERKARTUSCHE FÜR VERNEBLER**
TWO CHAMBER CARTRIDGE FOR ATOMIZERS
CARTOUCHE A DEUX CHAMBRES POUR ATOMISEUR

(30) Priorität: 17.10.1998 DE 19847968; 17.10.1998 DE 19847970; 14.12.1998 US 112380 P
(43) Veröffentlichungstag der Anmeldung: 01.08.2001
(60) Teilanmeldung: 02014579.3
(73) Patentinhaber: Boehringer Ingelheim Pharma KG, 55218 Ingelheim am Rhein (DE)
(72) Erfinder: HOCHRAINER, Dieter, D-55411 Bingen am Rhein (DE); ZIERENBERG, Bernd, D-55411 Bingen am Rhein (DE)
(74) Vertreter: Laudien, Dieter, Dr.
(86) Internationale Anmeldenummer: EP9907589
(87) Internationale Veröffentlichungsnummer: WO00023037

(56) Entgegenhaltungen:
- EP-A- 0 778 221
- DE-A- 19 615 422
- US-A- 2 362 103
- US-A- 3 842 836
- US-A- 4 727 985
- US-A- 5 533 994

## Beschreibung

Die vorliegende Erfindung betrifft eine Vorrichtung bestehend aus einer Verschlußkappe und einem Behälter in Form einer Zweikammer-Kartusche, in der ein Wirkstoff und ein Lösungsmittel bis zur Verwendung der Vorrichtung in einem Vernebler getrennt gelagert werden können, sowie ein treibgasfreies Wirkstoffkonzentrat, in der der Wirkstoff zum Zweck der Lagerung hochkonzentriert vorliegt. Die erfindungsgemäße Kartusche dient besonders als Container für Arzneimittelformulierungen zur Verwendung in Verneblern zur Erzeugung von Aerosolen für die inhalative oder nasale Applikation, insbesondere zur Verwendung in treibgasfreien Verneblern.

In der internationalen Patentanmeldung WO91/14468 "Atomizing Device and Methods" als auch in der WO 97/12687, dort Figuren 6a und 6b und der dazugehörigen Beschreibung, ist eine Vorrichtung zur treibgasfreien Verabreichung einer dosierten Menge eines flüssigen Arzneimittels zur inhalativen Anwendung beschrieben. In einem solchen Vernebler wird eine Arzneimittellösung mittels hohen Drucks in ein lungengängiges Aerosol überführt und versprüht. Für derartige Anwendungszwecke kann es erforderlich sein, die wirkstoffhaltigen Lösungen so in Behälter abzufüllen, daß nur geringe Luft- und Gasreste mit eingeschlossen werden. Dafür geeignete Behälter sind beispielsweise in der internationalen Patentanmeldung WO 96/06011 offenbart. Die dort beschriebenen Behälter sind vor allem für solche Arzneimittel geeignet, die in Form einer wäßrigen oder ethanolischen Lösung über einen längeren Zeitraum lagerstabil sind.

Um die Lagerfähigkeit von Wirkstoffen in Lösung, die sich bereits nach wenigen Monaten zersetzen, zu erhöhen, offenbart die DE 196 15 422 eine Kartusche, die zwei Kammern zur getrennten Aufbewahrung des Lösungsmittels und eines pulverförmigen oder als Tablette gepreßten Wirkstoffs aufweist. Die Kartusche ist derart gestaltet, daß beim Einsetzen der Kartusche in eine Vorrichtung zur Erzeugung des Aerosols (Inhalator) die Kammer, die den Wirkstoff enthält, mit einer Kanüle am Inhalator durchstochen wird, wobei der Wirkstoff mit dem Lösemittel im Behälter in Kontakt kommt und aufgelöst wird. Obgleich diese Kartusche zum Lagern von Inhalationsformulierungen in besagten Behältern viele Vorteile aufweist, kann mitunter die Kanüle des bestimmungsgemäßen Inhalators verstopfen, wenn sie durch die Kammer hindurchgestoßen wird, die den Wirkstoff enthält. Aus diesem Grund können in besagter Kammer auch keine Arzneimittelsuspensionen gelagert werden. Zusätzlich ist nicht immer zu gewährleisten, daß sich als Feststoffe formulierte Wirkstoffe ausreichend schnell in dem Lösungsmittel lösen, so daß die gewünschte Wirkstoffkonzentration nur unter zeitlicher Verzögerung erreicht wird. Dadurch kann ein reibungsloser Einsatz des mit der Kartusche beladenen Inhatators erschwert sein.

Die vorliegende Erfindung löst die Probleme der vorbeschriebenen und aus dem Stand der Technik bekannten Art, indem eine neue Zwei- oder Mehr-Kammer-Kartusche (Container) geschaffen wird, in der zwei oder mehr Bestandteile der Inhalationsformulierung getrennt voneinander aufbewahrt werden können.

Insbesondere betrifft die Erfindung eine Vorrichtung bestehend aus einer Verschlußkappe und einem Behälter, die als Kartusche (Container) für Vernebler eingesetzt werden kann. In der Kartusche kann mindestens ein Wirkstoff und ein Lösungsmittel einer zur inhalativen oder nasalen Applikation bestimmten therapeutischen Formulierung solange getrennt gelagert werden bis die Kartusche in den bestimmungsgemäßen Vernebler eingebaut wird. Als Vernebler sind besonders Hochdruckzerstäuber und insbesondere die Hochdruckzerstäuber der WO 91/14468 und der WO 97/12687, dort Figuren 6a und 6b und die dazugehörige Beschreibung, geeignet. Dabei ist die Kartusche so gestaltet, daß die getrennt gelagerten Bestandteile während des Einsetzen der Kartusche in den Inhalator, ohne ein Verstopfen der Kanüle zu verursachen, miteinander vermischt werden können, so daß sich innerhalb kürzester Zeit, bevorzugt innerhalb weniger Minuten und gegebenenfalls innerhalb weniger Sekunden, die einsatzfertige Arzneimittelzubereitung bildet. Im Kontext der vorliegenden Erfindung ist es unerheblich, ob die zu applizierende Arzneimittelformulierung eine Lösung oder eine Suspension ist, entscheidend ist, daß es sich bei der zu applizierenden Formulierung um eine flüssige Formulierung handelt, die mittels eines Verneblers der oben beschriebenen Art in ein Aerosol für die inhalative oder nasale Applikation überführt werden kann. Zur inhalativen Anwendung mittels des eingangs beschriebenen Inhalators werden im allgemeinen jedoch Lösungsformulierungen gegenüber Suspensionsformulierungen bevorzugt.

Im Rahmen dieser Beschreibung werden die Ausdrücke Vemebler, Zerstäuber, Inhalator und Hochdruckzerstäuber sowie Dosieraerosol und Aerosol gleichwertig nebeneinander als Synonyme benutzt, sofern nicht extra gekennzeichnet.

### Beschreibung der Erfindung

Die vorliegende Erfindung betrifft eine Vorrichtung nach Anspruch 1, sowie die Verwendung dieser Vorrichtung wie im Anspruch 29 definiert.

Die vorrichtung ist ähnlich der Erfindung der bereits erwähnten DE 196 15 422 aufgebaut. Wie in der DE 196 15 422 besitzt die erfindungsgemäße Kartusche wenigstens zwei Möglichkeiten zum Aufbewahren von Formulierungen bzw. Formulierungsbestandteilen: den Behälter der Kartusche und eine weitere Kammer, die vorzugsweise in der Verschlußkappe der Kartusche ausgebildet ist. Einige Bestandteile der zu applizierenden Formulierung werden bis zur Verwendung in besagter Kammer gelagert, die restlichen Bestandteile in dem Behälter, darunter der größte Teil der flüssigen Komponente. Diese flüssige Komponente beinhaltet das für die Applikation vorgesehene Lösungs- oder Suspensionsmittel, bzw. den größten Teil davon, bevorzugt ein Lösungsmittel und ggf. weitere Komponenten auf die im Speziellen weiter unten eingegangen wird. Unter dem Begriff "den größten Teil davon" wird der Teil der zu applizierenden Formulierung verstanden, der nicht bereits zusammen mit dem Wirkstoff in der Kammer vorliegt. Im Folgenden wird nicht streng zwischen Lösungsmittel und Lösungsmittelgemisch unterschieden, d.h. der Begriff Lösungsmittel schließt ein Lösungsmittelgemisch mit ein, sofern nicht anders gekennzeichnet. Umgekehrt besteht ein Lösungsmittelgemisch jedoch immer aus mindestens zwei flüssigen, chemisch unterschiedlichen Komponenten.

In der erwähnten Kammer können Wirkstoffe pharmazeutisch stabil über einen längeren Zeitraum von beispielsweise mehreren Monaten oder gegebenenfalls Jahren gelagert werden, insbesondere Wirkstoffe, die in ethanolischen Lösungen nicht über den besagten Zeitraum pharmazeutisch stabil gelagert werden können. Die Kammer ist bevorzugt in der Verschlußkappe ausgebildet, kann sich jedoch auch an einer anderen Stelle der Kartusche, z.B. im Inneren des Behälters befinden.

Aus Gründen der Klarheit wird im weiteren Verlauf dieser Beschreibung der Ausdruck Kammer sowohl für eine Kammer als auch mehrere Kammern gleicher Gestaltung und in unmittelbarere Nachbarschaft zueinander benutzt, sofern nicht anders gekennzeichnet.

Die Kammer weist wenigstens eine, bevorzugt jedoch zwei Öffnungen aus. Die Verschlußkappe ist so geschaffen, daß der Inhalt der Kammer durch die eine, in jedem Fall ausgebildete Öffnung durch eine äußere Einwirkung in das Innere des Behälters überführt werden kann, auch wenn die Verschlußkappe den Behälter fest verschließt. Diese Öffnung wird im folgenden kurz Innenöffnung genannt.

Daneben kann die Kammer optional eine weitere Öffnung aufweisen. Über diese Öffnung kann im geschlossenen Zustand der Kartusche eine Verbindung zwischen Kammer und Außenumgebung hergestellt werden, so daß z.B. über sie ein Gegenstand, wie z.B. ein Kolben, ein Stopfen, eine Kanüle, eine Kapillare oder ein Stift von außerhalb in die Kammer hinein befördert werden kann. Diese Öffnung wird im Folgenden Außenöffnung genannt. Es sei ausdrücklich darauf hingewiesen, daß die Begriffe "Innenöffnung" und "Außenöffnung" ausschließlich der begrifflichen Unterscheidung dienen und keine unmittelbaren Rückschlüsse auf die Lage oder die gegenseitige Orientierung der Öffnungen zulassen. Gegebenenfalls kann die Innenöffnung auch die Funktion der Außenöffnung übernehmen.

In einer bevorzugten Ausführungsform zeichnet sich die Kammer dadurch aus, daß wenigstens eine der Öffnungen, (bzw. in Varianten mit nur einer Öffnung, dieselbe), mit einem beweglichen und die Öffnung dicht verschließenden Kolben verschlossen ist, der zum Öffnen der Kammer durch eine externe Kanüle oder einen externen Stift in die Kammer hinein oder heraus bewegt werden kann.

Die Innenöffnung der Kammer kann entweder durch einen beweglichen Stopfen, durch eine durchstoßbare, leicht abreißbare Membran oder ein anderes bewegliches Dichtungselement verschlossen sein. Die optional ausgebildete Außenöffnung ist durch einen Kolben verschlossen, der derart dimensioniert ist, daß er die Kammer zum einen dicht verschließt, zum anderen jedoch unter Aufwendung einer Kraft in die Kammer hinein bewegt werden kann.

In einigen Ausführungsformen kann dieser Kolben innen hohl sein und eine Öffnung aufweisen. In solchen Varianten kann der Kolben die gesamte Kammer passgenau ausfüllen und in seinem Inneren befindet sich dann der Wirkstoff. Gegebenenfalls ist der Kolben so angeordnet, daß er gleichzeitig die Außenöffnung und Innenöffnung dicht verschließt und keine weiteren Dichtungselemente mehr notwendig sind, um den Wirkstoff in dem Hohlraum des Kolbens dicht verschlossen zu lagern.

Wird die Kartusche in den Inhalator eingesetzt, durchdringt eine am Inhalator ausgebildete Kanüle oder Stift die Verschlußkappe und öffnet dadurch direkt oder indirekt das Dichtungselement der Innenöffnung. Dies kann z.B. dadurch erreicht werden, daß die Kanüle das Dichtungselement bevorzugt ausgehend von einer Stelle außerhalb der Kammer aufreißt, durchstößt, öffnet oder wegbewegt. Gegebenenfalls kann die Kanüle das Dichtungselement, aber auch von einer Stelle innerhalb der Kammer öffnen. Das Dichtungselement kann z.B. eine Siegelfolie sein.

Alternativ kann die Kanüle den Kolben beim Einstecken der Kartusche in den Inhalator in die Kammer hinein drücken, so daß dadurch die Innenöffnung geöffnet wird, z.B. in dem das Dichtungselement entweder direkt von dem Kolben eingerissen, aufgerissen oder geöffnet wird oder es sich aufgrund des in der Kammer aufgebauten Überdrucks öffnet. Der Kolben wird dabei von der Kanüle entweder ganz durch die Kammer geschoben bis er durch die Innenöffnung in den Behälter fällt oder er verbleibt derart in der Kammer, daß er bei der Entnahme von Flüssigkeit durch die Kanüle nicht stört.

Anstelle des die Außenöffnung verschließenden Kolbens kann (können) auch ein oder mehrere andere Äquivalente zur Lösung der diesem Element zugrunde liegenden Aufgabe herangezogen werden. Dazu zählen Stopfen, Kugeln, Stacheln, Plättchen oder Abdichtungen verschiedener Art wie z.B. Siegelfolien, Klemm-, Steckoder Schraubverschlüsse und dergleichen.

Die erfindungsgemäße Vorrichtung kann als Kartusche für Inhalatoren sowohl für Monopräparate, als auch für Kombinationspräparate verwendet werden.
Im Fall von Monopräparaten wird der Wirkstoff bevorzugt in einer stabilen Formulierung in der Kammer aufbewahrt, die weiteren Bestandteile der zu applizierenden Arzneimittelzubereitung, darunter der größte Teil der flüssigen Komponente, in dem Behälter.
Im Fall von Kombinationspräparaten, können die aktiven Bestandteile als stabile Formulierung entweder in einer einzigen Kammer aufbewahrt werden oder in verschiedenen. Sind die verwendeten Wirkstoffe von deutlich unterschiedlicher Lagerstabilität, kann der empfindlichere Wirkstoff wie oben beschrieben in der Kammer aufbewahrt werden. Der andere, stabilere Wirkstoff kann zusammen mit dem in dem Behälter befindlichen Lösungs- oder Suspensionsmittel gelagert werden. Das setzt natürlich voraus, daß letzterer über die angestrebte Lagerzeit von mehreren Monaten oder Jahren in dem Lösungsmittel stabil ist.

Dadurch, daß empfindliche Substanzen in einer anderen als der zu applizierenden Formulierung solange aufbewahrt werden können, bis die Kartusche in den Inhalator eingebracht wird, kann die Lagerzeit der so gefüllten Kartusche gegenüber einer mit der fertigen Arzneimittelzubereitung wesentlich verlängert werden. Ein Wirkstoff kann in der Kammer als Pulver, Granulat, in Form einer Tablette, Lösung oder als Suspension aufbewahrt werden. Im Allgemeinen werden zur Lagerung des oder der Stoffe in der Kammer galenische Formulierungen bevorzugt, die nach dem Zusammenführen mit dem Lösungsmittel ein einfaches und schnelles Auflösen des Wirkstoffs in dem Lösungsmittel fördern. Bevorzugt sind Wirkstoffkonzentrate, die einen weiteren Aspekt der vorliegenden Erfindung darstellen.

Die erfindunggemäße Wirkstoffkonzentrat enthält einen oder mehrere Wirkstoffe, die inhalaltiv applizierbar sind und bevorzug für die inhalative Therapie eingesetz werden können. Daher betrifft die Erfindung auch die Verwendung eines solchen Wirkstoffkonzentrats in der Inhalationstherapie.

Das erfidnungsgemäße Wirkstoffkonzentrat kann durch Verdünnen mit einer pharmakoloigsch verträglichen Flüssigkeit, die gegebenenfalls pharmazeutische Hilfs- und Zusatzstoffe enthält, in eine Arzneimittelzubereitung (Aerosolformulierung) überführt werden, die unter Zuhilfenahme eines Vemeblers in ein inhalierbares Aerosol überführt wird. Dieses Verdünnungsmittel befindet sich bei Verwendung der erfindungsgemäßen Kartusche bevorzugt in dem Behälter (2). Dabei bestimmt die zu applizierende Arzneimittelzubereitung zusammen mit dem Wirkstoffkonzentrat die genaue Zusammensetzung des Verdünnungsmittels.

Das erfindungsgemäße Wirkstoffkonzentrat betrifft Lösungen oder Suspensionen, in denen der Wirkstoff hochkonzentriert in einer pharmakologisch geeigneten Flüssigkeit gelöst oder suspendiert vorliegt und die sich dadurch auszeichnen, daß darin der Wirkstoff über einen Zeitraum von mehreren Monaten gegebenefalls bis zu mehreren Jahren gelagert werden kann, ohne daß die pharmazeutische Qualität beeinträchtigt wird.

Unter dem Begriff "Wirkstoffkonzentrat" wird eine Lösung oder Suspension eines oder mehrerer Wirkstoffs (Wirkstoffe) verstanden, der (die) hochkonzentriert in einer pharmakolgisch verträglichen Flüssigkeit als Lösung oder Suspension vorliegt (vorliegen). Bevorzugt sind Suspensionen, da diese sich als besonders lagerstabil erwiesen.

Unter "hochkonzentriert" wird eine Konzentration des Wirkstoffs verstanden, die üblicherweise zu hoch ist, um die entsprechende Lösung oder Suspension ohne Verdünnung in therapeutisch vertretbarer Weise für inhalative Zwecke einsetzen zu können. In dem Wirkstoffkonzentrat kann die Konzentration des Wirkstoffs (der Wirkstoffe) die Konzentration der zu applizierenden Arzneimittelzubereitung um das 10-fache bis das 500-fache übersteigen, bevorzugte um das 100- bis 400-fache, insbesondere bevorzugt um das 250-350-fache. Erfindungsgemäß liegt die Wirkstoff-Konzentration in dem Wirkstoffkonzentrat für Suspensionen zwischen 10 mg/ml und 1000 mg/ml, bevorzugt zwischen 75 mg/ml und 1000 mg/ml, besonders bevorzugt zwischen 75 mg/ml und 500 mg/ml, insbesondere bevorzugt zwischen 100 mg/ml und 400 mg/ml und ganz besonders bevorzugt zwischen 250 mg/ml und 350 mg/ml. Bei Lösungen liegt der Konzentrationsbereich bevorzugt 10 mg/ml und 500 mg/ml, besonders bevorzugt zwischen 75 mg/ml und 500 mg/ml, insbesondere bevorzugt zwischen 75 mg/ml und 200 mg/ml und ganz besonders bevorzugt zwischen 75 mg/ml und 150 mg/ml. So kann z.B. Formoterol in einer Ausführungsform der erfindungsgemäßen Formulierung in einer Konzentration zwischen 10 mg/ml und 500 mg/ml, bevorzugt zwischen 75 mg/ml und 500 mg/ml, insbesondere bevorzugt zwischen 100 mg/ml und 400 mg/ml und ganz besonders bevorzugt zwischen 250 mg/ml und 350 mg/ml vorliegen. Zum Zweck der Inhalation muß diese Formulierung dann auf eine Konzentration von ca. 0,9 mg/ml bis 1,5 mg/ml verdünnt werden. Die Konzentrationsangaben beziehen sich auf mg freie Base Formoterol pro ml Wirkstoffkonzentrat.

Als Wirkstoff(e) können alle Substanzen verwendet werden, die für die inhalative Anwendung geeignet sind und in dem vorgegebenen Lösungs- oder Suspensionsmittel löslich sind oder suspendiert werden können. Bei den bevorzugten Wirkstoffen handelt es sich insbesondere um Betamimetica, Anticholinergika, Antiallergika, Leukotrien-Antagonisten und insbesondere um Steroide sowie Wirkstoffkombinationen davon.

### Im einzelnen seien als Beispiele genannt:

### Tiotropiumbromid, 3-[(hydroxydi-2-thienylacetyl)oxy]-8,8-dimethyl-,8-azoniabicyclo[3.2.1]oct-6-en-bromid

| Als Betamimetika: | | | |
|---|---|---|---|
| Bambuterol | Bitolterol | Carbuterol | Formoterol |
| Clenbuterol | Fenoterol | Hexoprenalin | Procaterol |
| Ibuterol | Pirbuterol | Salmeterol | Tulobuterol |
| Reproterol | Salbutamol | Sulfonterol | Terbutalin |

1-(2-Fluor-4-hydroxyphenyl)-2-[4-(1-benzimidazolyl)-2-methyl-2-butylamino]ethanol, erythro-5'-Hydroxy-8'-(1-hydroxy-2-isopropylaminobutyl)-2H-1,4-benzoxazin-3-(4H)-on,
1-(4-Amino-3-chlor-5-trifluormethylphenyi)-2-tert.-butyl-amino)ethanol, 1-(4-Ethoxycarbonylamino-3-cyan-5-fluorphenyl)-2-(tert.-butylamino)ethanol.

Als Anticholinergika:
Ipratropiumbromid
Oxitropiumbromid
Tropiumchlorid
Benzilsäure-N-β-fluorethylnortropinestermethobromid

### Als Antiallergika:

Dinatriumcromoglicat
Nedocromil
Epinastin

| Als Steroide: | |
|---|---|
| Flunisolid | Dexamethason-21-isonicotinat |
| Seratrodast | Mycophenolate mofetil |
| Pranlukast | Zileuton |
| Butixocort | Budesonid |
| Deflazacort | Budesonid |
| Fluticason | Promedrol |
| Mometason furoat | Tipredan |
| Beclometason (bzw. das 17,21-Dipropionat) | |
| Beclomethason, Douglas | Icomethason enbutat |
| Ciclometason | Cloprednol |
| Fluocortin butyl | Halometason |
| Deflazacort | Alclometason |
| Ciclometason | Alisactid |
| Prednicarbat | Hydrocortison-butyratpropionat |
| Tixocortol-pivalat | Alclometason-dipropionat |
| Lotrison | Canesten-HC |
| Deprodon | Fluticason-propionat |
| Methylprednisolon-Aceponat | Halopredon-acetat |
| Mometason | Mometasone-furoat |
| Hydrocortison-aceponat | Mometason |
| Ulobetasol-propionat | Aminoglutethimid |
| Triamcinolon | Hydrocortison |
| Meprednison | Fluorometholon |
| Dexamethason | Betamethason |
| Medryson | Fluclorolon acetonid |
| Fluocinolon acetonid | Paramethason-acetat |
| Deprodon Propionat | Aristocort-diacetat |
| Fluocinonid | Mazipredon |
| Difluprednat | Betamethason valerat |
| Dexamethasonisonicotinat | Beclomethason-Dipropionat |
| Fluocortoloncapronat | Formocortal |
| Triamcinolon-Hexacetonid | Cloprednol |
| Formebolon | Clobetason |
| Endrisone | Flunisolid |
| Halcinonid | Fluazacort |
| Clobetasol | Hydrocortison-17-Butyrat |
| Diflorason | Fluocortin |
| Amcinonid | Betamethason Dipropionat |
| Cortivazol | Betamethasonadamantoat |
| Fluodexan | Trilostan |
| Budesonid | Clobetason |
| Demetex | Trimacinolon Benetonid |

9.alpha.-chloro-6.alpha.-fluoro-11.beta.17.alpha.-dihydroxy-16.alpha.-methyl-3-oxo-1,4-androstadien-17.beta.-carboxysäure-methylester-17-propionat.

Salbutamol, Tiotropium und / oder Formoterol und deren Salze, insbesondere Formoterol, werden in dem Konzentrat bevorzugt als Suspensionen formuliert.

Unter dem Begriff "pharmakologisch geeignete Flüssigkeit" wird im Sinn der vorliegenden Erfindung ein Lösungs- oder Suspensionsmittel verstanden, das kein verflüssigtes Treibgas ist. Bevorzugt sind polare Flüssigkeiten, insbesondere bevorzugt sind protische Flüssigkeiten.

Beispiele für polare Lösungs- oder Suspensionsmittel für das Wirkstoffkonzentrat sind z.B. Dimethylsulfoxid oder Verbindungen, die Hydroxylgruppen oder andere polare Gruppen enthalten, beispielsweise Wasser oder Alkohole - insbesondere Ethanol, Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester u.ä..

Beispiele für protische Flüssigkeiten, die im Kontext der Erfindung die am stärksten bevorzugten Lösungs- bzw. Suspensionsmittel sind, sind Wasser, wäßrige Salzlösungen mit einem oder mehreren pharmakologisch verträglichen Salz(en), Ethanol oder einem Gemisch daraus.
Im Fall von wäßrigen Ethanol- Gemischen liegt das Volumenverhältnis Ethanol zu Wasser bzw. zur wäßrigen Salzlösung zwischen 5:95 und 99:1, bevorzugt zwischen 40:60 und 96:4, insbesondere bevorzugt zwischen 75:25 und 96:4. Ein besonders bevorzugtes Verhältnis liegt zwischen 40:60 und 60:40.

Für eine Salzlösung als Lösungs- oder Suspensionsmittel oder als Bestandteil davon besonders gut geeignete Salze sind solche die nach Applikation keine oder nur eine vemachlässigbar geringe pharmakologische Wirkung entfalten. Salzlösungen werden bevorzugt bei Suspensionskonzentraten eingesetzt. Durch den Salzzusatz wird das Lösungsvermögen von Wasser für den (die) Wirkstoff(e) deutlich reduziert, so daß ein die suspendierten Teilchen stabilisierender Effekt erzielt wird. Gegebenenfalls können gesättigte Salzlösungen verwendet werden. Die Menge an Salz hängt dabei von der exakten Zusammensetzung des Lösungs- oder Suspensionsmittels ab und seinem Vermögen den Wirkstoff zu lösen. Z.B. sollte in einer wäßrige Suspensionen von Formoterol im Sinn des erfindungsgemäßen Wirkstoffkonzentrats Formoterol zu weniger als 0,5% gelöst vorliegen, bevorzugt zu weniger als 0,1 %, wobei sich die Angaben auf die Gesamtmenge (Gewicht) an Formoterol beziehen. Liegt die gelöste Menge dennoch über den angegebenen Werten, kann es durch Zugabe von Salz unterhalb dieser Werte gesenkt werden.

In der Regel kann die Löslichkeit durch Salzzugabe auf die Hälfte gesenkt werden, in manchen Fällen auf ein Fünftel oder noch niedriger.
Bevorzugt sind Salzlösungen mit einem Salzgehalt von bis zu 50 Gew%, insbesondere bevorzugt bis zu 20 Gew.%.

Als Salze können sowohl anorganische als auch organische Salze verwendet werden. Bevorzugt sind anorganische Salze wie Natriumchlorid, Alkali- oder Ammonium-Halogensalze. Besonders bevorzugt ist Natriumchlorid. Als organische Salze eignen sich beispielsweise die Natrium-, Kalium- oder Ammoniumsalze der folgenden Säuren: Ascorbinsäure, Zitronensäure, Apfelsäure, Weinsäure, Maleinsäure, Bernsteinsäure, Fumarsäure, Essigsäure, Ameisensäure und/oder Propionsäure.

Dem Lösungs- oder Suspensionsmittel können Co-Solventien zugesetzt werden. Co-Solventien sind dazu geeignet, die Löslichkeit von Hilfsstoffen und gegebenenfalls des Wirkstoffs (der Wirkstoffe) zu erhöhen.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, sofern sie nicht bereits das Lösungs- oder Suspensionsmittel sind.

Dem erfindungsgemäßen Wirkstoffkonzentrat können auch weitere Hilfs- und Zusatzstoffe zugesetzt werden.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem (den) Wirkstoff(en) in dem pharmakologisch geeigneten Lösungs- oder Suspenionsmittel formuliert werden kann, um die qualitativen Eigenschaften des Wirkstoffkonzentrats oder der durch Verdünnung erhaltbaren, für inhalative Applikation geeigneten Arzneimittelzubereitung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe zur Stabilisierung von Suspensionen, sonstige Stabilisatoren, Komplexbildner, Antioxidantien und/oder Konservierungsstoffe, die die Verwendungsdauer der fertigen Arzneimittelformulierung verlängern, Geschmackstoffe, Vitamine und/oder sonstige dem Stand der Technik bekannte Zusatzstoffe.

Als oberflächenaktive Substanzen kann das Wirkstoffkonzentrat beispielsweise Sojalecithin, Ölsäure, Sorbitanester, wie Sorbitantrioleat oder andere aus dem Stand der Technik bekannte oberflächenaktive Stoffe (Surfactants) in der üblichen Konzentration enthalten.

Es hat sich gezeigt, daß der Zusatz einer organischen oder anorganischen Säure, bevorzugt in Kombination mit einem Komplexbildner, zu einer Verbesserung der Stabilität (Lagerstabilität) von einigen Arzneimitteln führt, die Ethanol als Lösungsmittel enthalten, insbesondere steroidhaltigen Arzneimitteln. Dies gilt im Besonderen für Arzneimittelzubereitungen, die Formoterol, Flunisolid bzw. sein Hydrat oder Hemihydrat oder Budenosid als Wirkstoff enthalten.

Beispiele für diesbezüglich bevorzugte anorganische Säuren sind: Salzsäure, Salpetersäure, Schwefelsäure und/oder Phosphorsäure.
Beispiele für besonders geeignete organische Säuren sind: Ascorbinsäure, Zitronensäure, Apfelsäure, Weinsäure, Maleinsäure, Bersteinsäure, Fumarsäure, Essigsäure, Ameisensäure und/oder Propionsäure u. andere. Bevorzugte Säuren sind Salzsäure und/oder Fumarsäure

Die Konzentration der Säure wird so gewählt, daß das Wirkstoffkonzentrat einen pH-Wert von 2,0 bis 7,0, bevorzugt zwischen 4,0 und 6,0 und ganz bevorzugt zwischen 4,5 und 5,5 aufweist.

Als Komplexbildner, die allein oder in Kombination mit einer Säure verwendet werden können, sind beispielsweise EDTA (Ethylendiamintetraessigsäure, bzw. ein Salz davon, wie beispielsweise das Dinatriumsalz), Zitronensäure, Nitrilotriessigsäure und deren Salze. Bevorzugter Komplexbildner ist EDTA.

Konservierungsstoffe können eingesetzt werden, um das Konzentrat vor Kontamination mit pathogenen Keimen zu schützen. Als Konservierungsstoffe eigenen sich die dem Stand der Technik bekannten, insbesondere Benzalkoniumchlorid oder Benzoesäure bzw. Benzoate wie Natriumbenzoat.

Als Antioxidantien eignen sich die bekannten pharmakologisch verträglichen Antioxidantien, besonders Vitamine oder Provitamine wie sie im meschlichen Organismus vorkommen, beispielsweise Ascorbinsäure oder Vitamin E.

Liegt der Wirkstoff oder liegen die Wirkstoffe in dem erfindungsgemäßen Wirkstoffkonzentrat als Suspension vor, werden die Teilchen bevorzugt in einer Partikelgröße von bis zu 20 µm formuliert, bevorzugt bis zu 10 µm und insbesondere bevorzugt bis zu 5 µm.

Bevorzugte Wirkstoffkonzentrate enthalten nur einen oder zwei Wirkstoff(e), besonders bevorzugt sind Wirkstoffkonzentrate mit einem Wirkstoff.

Suspensionen sind als Wirkstoffkonzentrat am stärksten bevorzugt.

Das erfindungsgemäße Wirkstoffkonzentrat hat den Vorteil, daß ein Wirkstoff so formuliert werden kann, daß er über einen längeren Zeitraum stabil bleibt. Dabei ist es nicht notwendig, daß das Konzentrat abgesehen von der Wirkstoffkonzentration der Zusammensetzung der fertigen Arzneimittelzubereitung entspricht. Zum Beispiel kann das Konzentrat in seinem pH-Wert erheblich von dem pH-Wert der zu applizierenden Arzneimittelzubereitung abweichen, wenn dadurch eine stabilere Lösung oder Suspension eines Wirkstoffs möglich wird.

Das erfindungsgemäße Wirkstoffkonzentrat ist als solches üblicherweise nicht direkt zur medizinischen Verwendung, insbesondere zur inhalativen Applikation geeignet. Wie bereits geschildert, besteht eine Verwendung des Wirkstoffkonzentrats darin, es in eine Arzneimittelzubereitung (Aerosolformulierung) zu überführen. Dabei wird unter dem Begriff "Arzneimittelzubereitung" eine Formulierung eines Arzneimittels verstanden, die zur inhalativen Applikation geeignet ist und bei der ein Arzneimittel oder Arzneimittelgemisch in der notwendigen und / oder empfohlenen Konzentration appliziert werden kann (können).

Die Arzneimittelzubereitung ist bevorzugt derart, daß sie die mittels eines geeigneten Verneblers inhalativ applizierbar ist.

Eine bevorzugte Maßnahme zur Überführung des Wirkstoffkonzentrats in eine applizierbare Arzneimittelzubereitung, stellt das Verdünnen des erfindungsgemäßen Wirkstoffkonzentrats durch ein pharmakologisch geeignetes Lösungs- oder Suspensionsmittel dar.

Um die zu applizierende Arzneimittelzubereitung zu erhalten, wird das Wirkstoffkonzentrat soweit verdünnt, daß die inhalierfertige Arzneimittelzubereitung entsteht. Die zu applizierende Arzneimittelzubereitung bestimmt zusammen mit dem Wirkstoffkonzentrat in der Kammer (17) die genaue Zusammensetzung des Verdünnungsmittels in dem Behälter (2).

Beispiele für Formulierungen, die zur Applikation geeignet sind, werden in der WO 97/01329 offenbart. Sollen solche Formulierungen im Kontext der vorliegende Erfindung appliziert werden, sind das Wikstoffkonzentrat in der Kammer (17) und das Verdünnungsmittel im Behälter (2) so zu wählen, daß sie, wenn sie miteinander vermischt sind, eine Formulierung gemäß oder analog der WO 97/01329 bilden.

In einer solchen applizierbaren Formulierung liegt der Anteil des gelösten Arzneistoffes an der fertigen Arzneimittelzubereitung im allgemeinen zwischen 0.001 und 5 % - vorzugsweise zwischen 0.05 und 3%, insbesondere 0.01 und 2%, wobei sich die Angaben auf Gew. % beziehen. Im Fall von Lösungen als fertige Arzneimittelzubereitung ist die maximale Konzentration des Arzneistoffes abhängig von der Löslichkeit im Lösungsmittel und von der erforderlichen Dosierung zur Erzielung der gewünschten therapeutischen Wirkung.

Als Lösungs- oder Suspenionsmittel für die fertige, d.h. für die inhalative oder nasale Applikation geeignete Arzneimittelzubereitung im Sinne der vorliegenden Erfindung werden u.a. wäßrige Lösungen oder wäßrige Salzlösungen, bevorzugt wäßrige Lösungen, angestrebt, die Ethanol enthalten können. Bevorzugt sind Lösungen mit mindestens 30% (v/v) Ethanol, besonders bevorzugt mit mindestens 50 % (v/v). Insbesondere bevorzugt sind Lösungen mit einem Ethanolgehalt von über 95 % (v/v). Die Konzentrationsangabe erfolgt in Volumen Prozent (v/v), wobei der Rest Wasser bzw. die wäßrige Salzlösung ist. Ganz besonders bevorzugt ist Ethanol, das bereits geringe Mengen Wasser enthält - beispielsweise 96% Ethanol, 4% Wasser (v/v) - so daß es nicht mehr hygroskopisch ist und azeotrop verdampft.

Die zu applizierende Arzneimittelzubereitung bestimmt zusammen mit dem Wirkstoffkonzentrat der Kammer (17) die genaue Zusammensetzung des Verdünnungsmittels in dem Behälter (2).

Die einsatzfähige Arzneimittelformulierung wird bevorzugt erst mit dem Einfügen der Kartusche in den Inhalator fertiggestellt, da erst hierdurch die Bestandteile der Kammer (17) mit denen des Behälters (2) vermischt werden. An dieser Stelle sei angemerkt, daß die einzelnen Komponenten oder Inhaltsstoffe des Verdünnungsmittels so definiert sind, wie im Zusammenhang mit dem Wirkstoffkonzentrat angegeben, sofern diese Komponenten oder Inhaltsstoffe dort beschrieben wurden oder sofern es nicht anders gekennzeichnet ist.

Zum Zweck der Verdünnung bevorzugte Lösungs- oder Suspensionsmittel sind treibmittelfreie Flüssigkeiten, bevorzugt polare, insbesondere bevorzugt protische Flüssigkeiten.

Besonders bevorzugte Verdünnungsmittel sind Wasser, wäßrige Salzlösungen mit einem oder mehreren pharmakologisch verträglichen Salz(en), Ethanol oder einem Gemisch daraus, besonders bevorzugt sind Wasse-Ethanol-Gemische. Im Fall von wäßrigen Ethanol- Gemischen liegt das Volumenverhältnis Ethanol zu Wasser bzw. zur wäßrigen Salzlösung derart, daß die inhalierfertige Arzneimittelzubereitung eine Formulierung mit mindestens 30% (v/v) Ethanol, besonders bevorzugt mit mindestens 50 % (v/v). Insbesondere bevorzugt sind Arzneimittelzubereitungen mit einem Ethanolgehalt von über 95 % (v/v). Die Konzentrationsangabe erfolgt in Volumen Prozent (v/v), wobei der Rest Wasser bzw. die wäßrige Salzlösung ist. Ganz besonders bevorzugt ist Ethanol, das bereits geringe Mengen Wasser enthält - beispielsweise 96% (v/v) Ethanol - so daß es nicht mehr hygroskopisch ist und azeotrop verdampft.

Es ist weder selbstverständlich noch notwendig; daß das Verdünnungsmittel identisch mit dem Lösungs- oder Suspensionsmittel des Wirkstoffkonzentrats ist. Gegebenenfalls kann letzteres auch nur einen oder wenige Bestandteile des Verdünnungsmittels aufweisen.

Es sei an dieser Stelle ausdrücklich darauf hingewiesen, daß die bereits im Zusammenhang mit dem erfindungsgemäßen Wirkstoffkonzentrat erwähnten Co-Solventien und/oder Hilfs- oder Zusatzstoffe und/oder Wirkstoffe auch oder nur in dem Verdünnungsmittel gelöst oder suspendiert vorliegen können.

Bevorzugte Ausführungsformen des Verdünnungsmittels enthalten Konservierungsmittel und/oder Komplexbildner.

Gegebenenfalls kann in dem Verdünnungsmittel eine Puffersubstanz, wie z.B.
Trinatriumphosphat, Dinatriumhydrogenphosphat, Natriumdihydrogenphosphat, Na-EDTA, EDTA, Gemische daraus u.a. aus dem Stand der Technik bekannte Stoffe vorhanden sein. Bevorzugt sind Natriumdihydrogenphosphat, Dinatriumhydrogenphosphat, Trinatriumhydrogenphosphat, Kaliumdihydrogenphosphat, Kaliumhydrogenphosphat, Trikaliumhydrogenphosphat und Gemische daraus. Puffersubstanzen sind besonders dann von Vorteil, wenn das erfindungsgemäße lagerfähige Wirkstoffkonzentrat einen pH-Wert aufweist, der deutlich von dem für die Applikation gewünschten abweicht, z.B. wenn dies die Stabilität des Wirkstoffs während der Lagerung erhöht. In diesem Fall liegt die Puffersubstanz im Verdünnungsmittel in einer solchen Konzentration vor, daß nach Vermischen des Wirkstoffkonzentrats mit dem Verdünnungsmittel eine applikationsfähige Aerosolformulierung mit dem gewünschten pH-Wert, bevorzugt zwischen 2,0 und 7,0, besonders bevorzugt zwischen 4,0 und 6,0 und ganz besonders bevorzugt zwischen 4,5 und 5,5 vorliegt.

In einer bevorzugten Ausführungsform enthält die Arzneimittelzubereitung einen Komplexbildner, der bevorzugt aus einem im Zusammenhang mit dem Wirkstoffkonzentrat genannten Komplexbildner ausgewählt wird. Die Menge an Komplexbildner beträgt bis zu 100 mg/100 ml bevorzugt bis zu 50 mg /100ml. Bevorzugter Komplexbildner ist EDTA. Eine bevorzugte Arzneimittelzubereitung enthält beispielsweise in der zu applizierende Arzneimittelzubereitung 1,667 Gew.% Flunisolid, 0.01 mg/100 ml EDTA und Ethanol (96 % v/v) als Lösungsmittel. Durch Zusatz von Säure (Salzsäure) ist ein pH zwischen 3.0 und 4.0, bevorzugt 4.0 eingestellt.

Enthält das Verdünnungsmittel einen oder mehrere Wirkstoffe, so kann das Verdünnungsmittel zum Stabilisieren eine organischen oder anorganischen Säure, bevorzugt in Kombination mit einem Komplexbildner, enthalten, insbesondere bei steroidhaltigen Arzneimitteln. Dies gilt im Besonderen für Arzneimittelzubereitungen, die Formoterol, Flunisolid bzw. sein Hydrat oder Hemihydrat oder Budenosid als Wirkstoff enthalten.

Wie bereits erwähnt, bestimmt die zu applizierende Arzneimittelzubereitung zusammen mit dem Wirkstoffkonzentrat die genaue Zusammensetzung des Verdünnungsmittels.

Bevorzugte Arzneimittelzubereitungen enthalten einen oder zwei Wirkstoffe, besonders bevorzugt sind Arzneimittelzubereitungen mit einem Wirkstoff.

Weder das erfindungsgemäße lagerfähige Wirkstoffkonzentrat noch die durch Verdünnung erhaltene zu applizierende Arzneimittelzubereitung enthält ein Treibmittel.

Bevorzugt findet das Vermischen bei Umgebungstemperatur und Normaldruck statt. Ein Vorteil des erfindungsgemäßen Wirkstoffkonzentrats besteht darin, daß es sich durch Verdünnen innerhalb von sehr kurzer Zeit, beispielsweise innerhalb weniger Minuten oder gegebenenfalls innerhalb von wenigen Sekunden in eine therapeutisch wirksame und/oder zur Verwendung in einem Vernebler geeignete Formulierung überführen läßt. Dabei kann das Vermischen auch von Patienten, durchgeführt werden, die in der Regel kein pharmazeutisches Wissen besitzen.

Alternativ zu der bisher beschriebenen Aufteilung der einzelnen Bestandteile der zu applizierenden Arzneimittelzubereitung zum Zweck der Inhalation kann auch in dem Behälter (2) der Wirkstoff gelagert werden, bevorzugt als eine lagerfähige Lösung oder Suspension, die nicht notwendigerweise der zu applizierenden Arzneimitteluzubereitung entspricht. Die Konzentration der Wirkstoffe entspricht in diesem Fall bereits der Konzentration der oben beschriebenen zu applizierenden Arzneimittelzubereitung (plus/minus 10 Gew.%). Die Kammer (17) enthält dann die weiteren Hilfs- und Zusatzstoffe, bevorzugt die oben erwähnten, um die zu applizierende Arzneimittelzubereitung herzustellen. Diese können dann als Pulver, Tabletten, Suspensionen oder Lösungen vorliegen.

Beispielsweise kann ein Wirkstoff als Lösung oder Suspension in dem Behälter (2) bei einem pH-Wert aufbewahrt werden, der saurer oder basischer ist als derjenige der zu applizierenden Arzneimittelzubereitung. In diesem Fall enthält die Kammer (17) beispielsweise Puffersubstanzen, bevorzugt die oben beschriebenen. Beim Einstecken der Kartusche in den Inhalator wird dann der für die Applikation erwünschte pH-Wert durch Vermischen der Inhaltsstoffe der Kammer (17) mit der Formulierung in dem Behälter (2) hergestellt. Beispielsweise kann eine flüssige Formulierung mit Tiotropiumbromid als Wirkstoff in dem Behältnis (2) bei einem pH-Wert kleiner oder gleich 3,0 gelagert werden. In der Kammer (17) befinden sich dann Puffersubstanzen, wie z.B. die oben genannten, beispielsweise Natrium- und Kalium- Di- und Monohydrogenphosphate, in einer Menge, die gewährleistet, daß sich beim Vermischen dieser Puffersubstanzen mit der Tiotropium-Formulierung ein pH-Wert von 3,0 bis 7,0, bevorzugt 3,0 bis 4,0 einstellt. Diese Angaben stellen lediglich eine Auswahl der Formulierungsmöglichkeiten für Tiotropiumbromid dar und sind nicht notwendigerweise identisch für andere der erwähnten Wirkstoffe. Auch kann der Wirkstoff im Behälter (2) als Suspension gelagert werden und in der Kammer (17) ein pharmakologisch verträgliches Lösungsmittel, so daß beim Vermischen eine Lösungsformulierung entsteht.

Das bevorzugte Volumen der Formulierungen in der Kammer (17) und dem Behälter (2) wird maßgebliche durch die Volumina der Kammer (17) und des Behälters (2) vorgegeben. Zum Zweck der Lagerung in der oben erwähnten Kartusche wird in bevorzugten Ausführungsformen die Menge des erfindungsgemäßen, lagerfähigen Wirkstoffkonzentrats so gewählt, daß sie einem Volumen von 0,001 bis zu ca. 0,05 ml, bevorzugt von 0,001 bis zu 0,02 ml entspricht.

Die oben genannten Beispiele machen deutlich, daß in der vorliegenden Zwei-Kammer-Kartusche verschieden Komponenten einer zu applizierenden Wirkstoff-Formulierung bis zum Einsatz der Kartusche in den bestimmungsgemäßen Inhalator getrennt gelagert werden können. Dabei ist es nicht notwendig, daß die so gelagerten Komponenten für sich applikationsfähig sind. Wesentlich ist nur, daß die so gelagerten Komponenten beim Einsatz der Kartusche in den bestimmungsgemäßen Inhalator so miteinander vermischt werden, daß dabei die gewünschte, zu applizierende Formulierung entsteht.

### Beschreibung der erfindungsgemäßen Kartusche im Detail

Nachfolgend wird die Erfindung unter Zuhilfenahme konkreter Ausführungsbeispiele näher erläutert.

Figur 1 zeigt einen axialen Schnitt entlang der Längsachse der erfindungsgemäßen Kartusche (1) bestehend aus einem Behälter (2) für das Lösungsmittel und einer Verschlußkappe (3), bei der im wesentlichen nur die Verschlußmerkmale aufgeführt sind. Die weiteren Charakteristika der Verschlußkappe (3) werden in den nachfolgenden Fig. 3 bis 11 näher beschrieben.

Figur 2 zeigt eine Ausführungsform der Verschlußkappe (3) mit alternativer Verschlußtechnik, wobei analog Fig. 1 nur die Verschlußmerkmale detailliert ausgeführt sind. Der Behälter (2) ist nur angedeutet.

Die Fig. 3 zeigt eine Ausführungsform der Verschlußkappe (3) mit Kammer (17), einem Kolben (21) und einer Führungsröhre (16) in axialem Schnitt.

Fig. 4a bis 4g zeigen verschiedene Ausführungsformen des Kolbens (21) in axialem Schnitt.

Fig. 5 zeigt eine Ausführungsform der Verschlußkappe (3) mit einem hohlen Kolben (21) in axialem Schnitt.

Fig. 6 zeigt eine weitere Ausführungsform der Verschlußkappe (3) mit einem hohlen Kolben (21) in axialem Schnitt.

Fig. 7 zeigt eine weitere Ausführungsform der Verschlußkappe (3) mit einem hohlen Kolben (21) in axialem Schnitt.

Fig. 8 zeigt eine weitere Ausführungsform der Verschlußkappe (3) mit einem hohlen Kolben (21) in axialem Schnitt.

Fig. 9 zeigt eine Ausführungsform der Verschlußkappe (3) mit einem Kolben (21), einer Führungsröhre (16) und einer Kammer (17), in der zwei Kompartimente ausgebildet sind, in axialem Schnitt.

Fig. 10 zeigt eine Ausführungsform der Verschlußkappe (3) ohne passgenaue Führungsschiene für die Kanüle (15) in axialem Schnitt.

Fig. 11 zeigt eine Ausführungsform der Verschlußkappe (3), bei der die Kammer (17) nur eine Öffnung aufweist, die mit einem Kolben (21) verschlossen ist.

Fig. 12 zeigt eine Ausführungsform mit einer abreißbaren Kammer (17) am Stutzen (7).

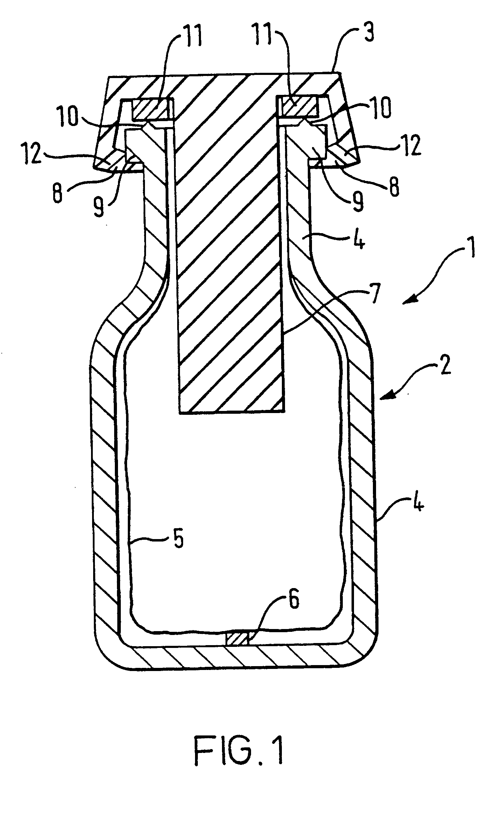

Figur 1 zeigt einen axialen Schnitt entlang der Längsachse der erfindungsgemäßen Kartusche (1), bestehend aus einem Behälter (2) für das Lösungsmittel und einer Verschlußkappe (3). In der Zeichnung sind nur solche Charakteristika der Verschlußkappe (3) aufgenommen, die zum Verschließen des Behälters (2) notwendig sind. Auf die weiteren Charakteristika der Verschlußkappe (3) wird erst in den nachfolgenden Figuren näher eingegangen.

Der erfindungsgemäße Behälter (2) besteht aus einem "Mantel" (4) und einem leicht verformbaren Innenbeutel (5). Der Innenbeutel (5) kleidet die Innenwandung des Mantels (4) aus, ist aber bis auf einen Bereich am Hals des Behälters (2) und dem Steg (6) nicht fest mit dem Mantel (4) verbunden. Der Vorteil des so befestigten, verformbaren Innenbeutels liegt darin, daß aus ihm eine Flüssigkeit über eine Kanüle entnommen werden kann, ohne daß sich in seinem Inneren ein Unterdruck aufbaut, der einer weiteren Entnahme entgegenwirkt oder daß der Innenbeutel während der Entnahme der Flüssigkeit unvorteilhaft zusammenfällt.
Im Folgenden, insbesondere in den nachfolgenden Patentansprüchen, wird nicht mehr zwischen "Behälter (2)", Mantel (4) und "Innenbeutel (5) " näher differenziert, sondern es wird ausschließlich der Begriff "Behälter (2)" verwandt.

Die Verschlußkappe (3) weist eine Einrichtung - hier in Form eines eintauchenden Stutzens (7) - auf, durch die während des Verschlußvorganges ein Teil des Inhalts aus dem Behälter (2) verdrängt wird. Bevorzugt ist der Stutzen (7) derart, daß erwenn die Verschlußkappe (3) den Hals des Behälters (2) fest verschließt - den Innenmantel des Halses des Behälters (2) nicht berührt oder nur so berührt, daß im geschlossenen Zustand der Kartusche eine Spalt zwischen dem Innenmantel des Behälters (2) und dem Stutzen (7) besteht, der erlaubt daß während des Verschlußvorgangs Gas oder Flüssigkeit aus dem Inneren des Behälters (2) entlang dem Stutzen entweichen kann. Alternativ kann der Stutzen (7) an seiner Oberfläche auch einen oder mehrere über die gesamte Länge des Stutzens (7) senkrecht verlaufende Einkerbungen aufweisen, über die während des Verschlußvorgangs Gas oder Flüssigkeit aus dem Inneren des Behälters (2) entweichen kann (nicht dargestellt). In diesem Fall kann der Außendurchmesser des Stutzens (7) dem Innendurchmesser der Behälterhalses entsprechen.
Im geschlossen Zustand verschließt die Kappe (3) den Behälter (2) dicht, so daß kein Gas oder keine Flüssigkeit mehr aus dem Inneren des Behälters (2) austreten kann.

Ein innenliegender umlaufender Wulst (8) am unteren Rand des Kopfes der Verschlußkappe (3) rastet in der Verschlußposition unterhalb eines an der Außenseite des Behälterhalses umlaufenden zylindrischen Ringes (9) ein. In der Verschlußposition wird der Zwischenraum zwischen dem flachen Teil der Verschlußkappe (3) und dem oberen Rand des Behälterhalses, der ggf. zur besseren Abdichtung mit einer umlaufenden Rippe (10) versehen ist, durch einen Dichtungsring (11) ausgefüllt und damit der Innenraum des Behälters (2) abgedichtet. Der Innendurchmesser des Dichtungsrings (11) wird zweckmäßig so gewählt, daß er an dem Stutzen (7) eng anliegt. Über eine oder mehrere Entlüftungsöffnung(en) (12) am Kopf der Verschlußkappe (3) kann das durch den Verschlußvorgang aus dem Inneren des Behälters (2) verdrängte Medium (Gas, Luft oder Flüssigkeit) entweichen. Die Entlüftungsöffnung(en) (12) kann (können) sich auch an anderen Stellen der Kartusche befinden, etwa seitlich in dem zylindrischen Teil der Kappe.

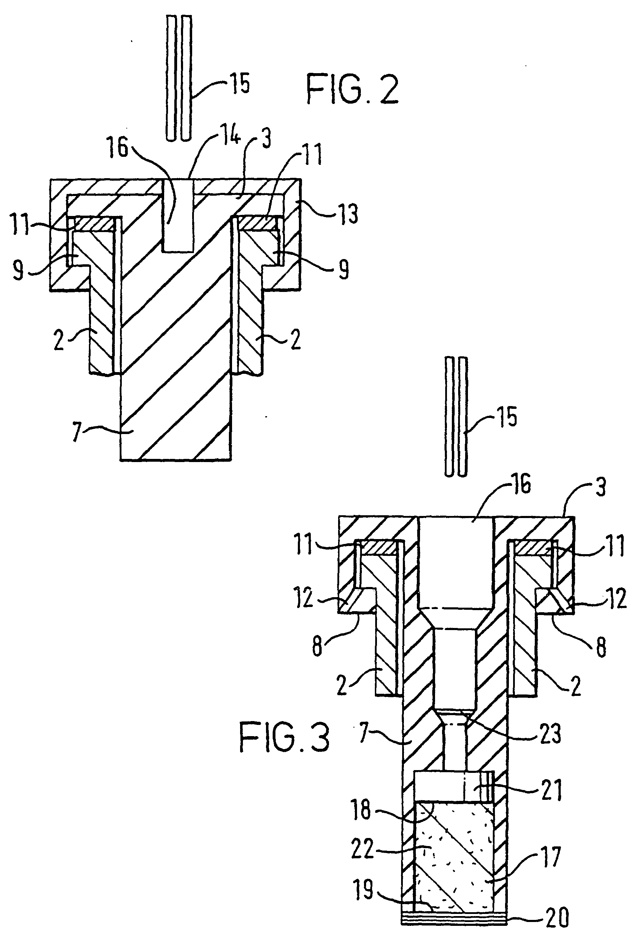

Fig. 2 zeigt eine Ausführungsform im axialen Querschnitt bei der die Verschlußkappe (3) durch eine Hülse (13) aus Aluminium, die gecrimpt wird, verschlossen wird. In der Zeichnung sind wieder nur die Charakteristika der Verschlußkappe (3) dargestellt, die zum Verschließen des Behälters (2) notwendig sind. Alle weiteren Charakteristika der Verschlußkappe (3) werden in den nachfolgenden Figuren näher erläutert. Der Behälter (2) ist nur angedeutet.

Die Hülse (13) ist derart gestaltet, daß sie eine zentrale Öffnung (14) zur Durchführung der Kanüle (15) aufweist. Die Fortsetzung der Öffnung (14) als Führungsrohr (16) für die Kanüle (15) durch die Verschlußkappe (3) ist nur angedeutet.
Die Öffnung (14) kann durch ein Septum als Originalitätsverschluß oder zum Schutz vor Staub und anderen Verunreinigungen verschlossen sein. Diese Verschlußtechnik ist beispielsweise bei Injektionsampullen bekannt. Ein derartiger Originalitätsverschluß kann auch in einer Verschlußkappe nach Fig. 1 ausgebildet sein.

In den nachfolgenden Figuren 3 bis 11 sind verschiedene Ausführungsformen der Verschlußkappe (3) im axialen Schnitt dargestellt. Die Kammer (17) ist als integraler Bestandteil des Stutzens (7) dargestellt. Der Behälter (2) ist nicht abgebildet. Alle Figuren zeigen die Verschlußkappe mit einem Verschlußsystem, wie es in Figur 1 dargestellt ist. Analoge Ausführungsformen können auch ein Verschlußsystem gemäß Fig. 2 aufweisen, sind jedoch nicht dargestellt.

Fig. 3 zeigt eine Ausführungsform einer Verschlußkappe (3) im axialen Schnitt. Zentrales Element der Verschlußkappe (3) ist eine Kammer (17), die mit dem Fuß des Stutzens (7) abschließt und den oder die oben beschriebenen Inhaltsstoff(e) (22) enthält, bei denen es sich z.B. um einen oder mehrere Wirkstoffe, Hilfsstoffe, Zusatzstoffe, handeln kann, die z.B. als Lösungs- oder Suspensionkonzentrat, als Pulver, Granulat oder einer anderen der oben beschriebenen Formen vorliegen können. Bevorzugt enthält die Kammer das oben beschriebene Wirkstoffkonzentrat. Die Kammer (17) weist zwei Öffnungen auf, (18) und (19). Durch die Öffnung (18) kann ein Gegenstand von außen in die Kammer (17) eingeführt werden, durch die Öffnung (19) kann der Inhalt der Kammer (17) in den Behälter (2) entleert werden. Die Öffnung (19) ist mit einer Abtrennung (20) verschlossen, die über diese hinausgeht und fest mit dem Stutzen (7) verbunden ist. Die Öffnung (18) ist durch einen Kolben (21) verschlossen. Von der Kopfseite der Verschlußkappe (3) führt eine Führungröhre (16) für eine Kapillare oder Kanüle (15) zur Entnahme von Flüssigkeit bis zu dem Kolben (21). In der Führungsröhre kann ein Septum oder eine O-Ring Dichtung (23) ausgebildet sein.

Die Position der Abtrennung (20) bzw. des Kolbens (21) kann in weiten Bereichen des Innenraums des Stutzens (7) variieren, bevorzugt ist sie jedoch in Abhängigkeit der Menge an Inhaltsstoff(en) (22) so angeordnet, daß der durch die Abtrennung (20) bzw. die Abtrennung ( 20) und den Kolben (21) gebildete Innenraum möglichst geringe Menge an Gasvolumen (Luft), bevorzugt keinerlei Gasvolumen, umfaßt.

Die Abtrennung (20) kann beispielsweise eine durch Druck einreißbare oder durch einen spitzen oder scharfen Gegenstand leicht aufreißbare Folie sein. Bevorzugt sind angeschweißte diffusionsdichte Siegelfolien. Derartige Siegelfolien können z.B. eine Aluminiumschicht enthalten. In einer Ausführungsform kann die Abtrennung (20) an ihrer Verbindung zur Seitenwand des Stutzens (7) Schwachstellen aufweisen, so daß sie an diesen Schwachstellen aufreißt, wenn auf sie ein Druck oder eine Kraft ausgeübt wird. Alternativ zu einer Siegelfolie kann die Öffnung (19) auch durch einen Stopfen oder Kolben verschlossen sein (nicht abgebildet).

Der Kolben (21) ist aus einem Material, das so hart ist, daß eine abgerundete Kanüle nicht hindurch stechen kann, bzw. so unelastisch, daß der Kolben nicht an der Kanüle haften bleibt, wenn die Kanüle fest auf ihn drückt. Bevorzugtes Material ist ein Elastomer, Weichplastik und/oder Kunststoffe wie Polyethylen, Silikon, EPDM (Ethylen-Propylen-Dien-Copolymer).

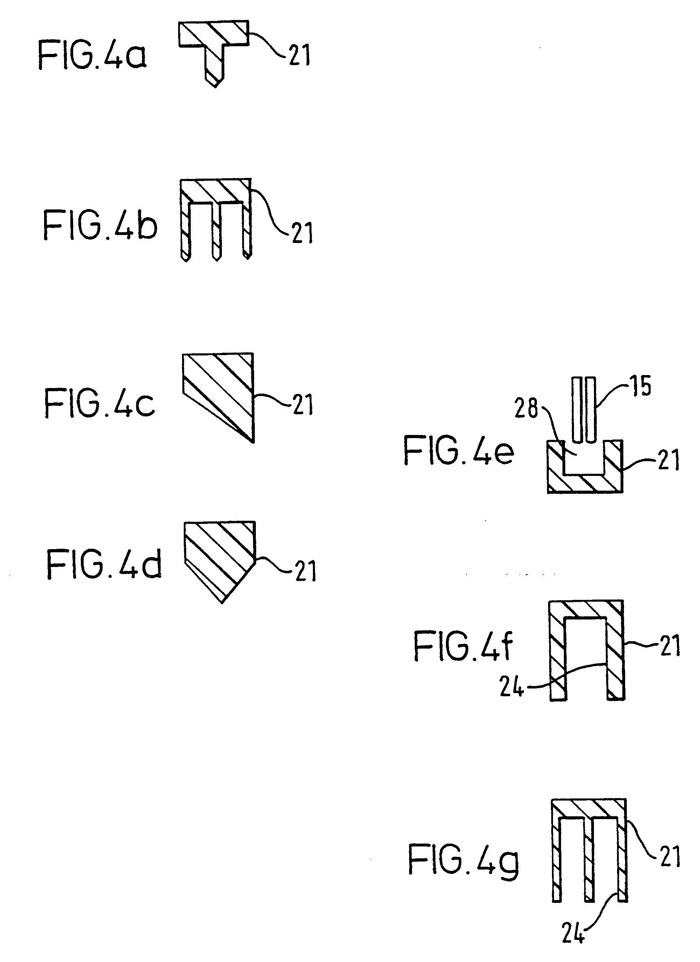

Die Form des Kolbens (21) kann z.B. zylinderartig sein. In jedem Fall ist er dergestalt, daß er die Öffnung, für die er vorgesehen ist, dicht verschließt. Bevorzugt weist der Kolben (21) eine Länge auf, die größer als der Durchmesser ist, z.B. um einen Faktor zwischen 1,2 bis 2,0, bevorzugt um den Faktor 1,5. Dadurch wird der Kolben (21), wenn er bewegt wird, entlang seiner Mantelfläche geführt und ein Querstellen verhindert. In Fig. 4a bis 4g sind weitere Ausführungsformen des Kolbens (21) dargestellt, bei denen eine oder mehrere Dorn(en) oder Schneid(en) (Fig. 4a und 4b) ausgebildet sind, bei denen eine Seite abgeschrägt ist (Fig. 4c) oder bei der der Kolben (21) spitz zuläuft (Fig. 4d). Die ausgebildeten Dornen oder Schneiden können von geringem Durchmesser sein (µm bis mm) und sind höchstens so lang, daß sie in der Ausgangsposition des Kolbens (21), d.h. bevor der Kolben (21) durch die Kanüle (15) bewegt wird, bis zur Abtrennung (20) reichen oder bis kurz davor.

Eine weitere bevorzugte Ausführungsform des Kolbens (21) ist in Figur 4e dargestellt. Der Kolben weist eine Aussparung (28) in Form eines Hohlraums auf, der nach einer Seite hin offen ist. Die Öffnung der Aussparung (28) weist zum Kopf der Verschlußkappe (3) hin, also in Richtung Kapillare oder Kanüle (15). Der Innendurchmesser der Öffnung bzw. der Aussparung (28) ist größer als der Außendurchmesser der Kanüle (15). Im Querschnitt hat der Kolben (21) die Form eines U mit ggf. eckig ausgebildeten Kanten. Der Boden der Aussparung (28) bildet die Stelle des Kolbens (21) an der die Kanüle (15) angreifen kann, um den Kolben (21) in oder durch die Kammer (17) zu drücken. Der Vorteil dieser Gestaltung und Anordnung besteht darin, daß sich der Kolben (21) durch den Druck der Kanüle (15) auf den Boden der Aussparung (28) an dem gegenüberliegenden Ende leicht verjüngen kann, also an der Seite des Kolbens (21), die die Öffnung (18) unmittelbar verschließt. D.h. durch das Drücken der Kanüle (15) geht im Querschnitt die Form des Kolbens (21) von der U-Form annäherungsweise in die eines V über. Dadurch wird eine vereinfachte Passage des Kolbens (21) durch die Öffnung (18) erreicht. Ein weiterer Vorteil dieser durch den Druck der Kanüle (15) verursachten Gestaltänderung des Kolbens (21) besteht darin, den Druck des Kolbens (21) auf die ihn haltenden Wände zu verringern, so daß selbst ein fest sitzender Kolben (21) gelöst und von der Kanüle (15) ohne Verkanten bewegt werden kann. Diese Ausführungsform ist besonders dann von Vorteil, wenn der Kolben (21) wie z.B. in Fig. 3 dargestellt, die Kammer (17) von innen gegen die Öffnung (18) verschließt.

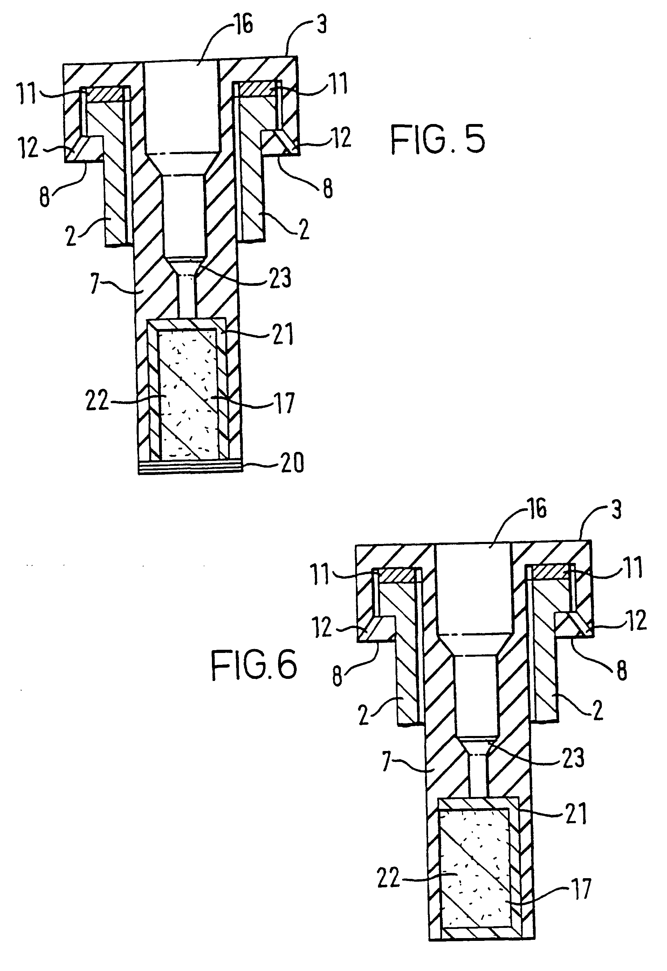

In Figur 4 f ist ein Kolben (21) mit einem Hohlraum (24) dargestellt, der passgenau oder annähernd passgenau die gesamte Kammer (17) der Länge und Breite nach ausfüllt. Unter passgenau wird in diesem Zusammenhang verstanden, daß der Kolben (21) die gleichen Außendimensionen aufweist wie das Innere der Kammer (17) oder gegebenenfalls um bis zu 5% breiter ist als die Kammer (17). Unter annähernd passgenau wird verstanden, daß der Kolben (21) im Durchmesser und/oder in der Länge geringfügig kleiner ist als das Innere der Kammer (17). Bevorzugt ist der Kolben (21) als passgenauer Kolben ausgebildet. Eine solche Variante kann beim Befüllen der Verschlußkappe (3) von Vorteil sein, aber auch beim Durchführen des Kolbens durch die Kammer (17) mittels der Kanüle (15). Der Kolben (21) kann dabei so in die Kammer eingebettet sein, daß die Öffnung des Hohlraums (24) durch die Abtrennung (20) verschlossen wird (Fig. 5), oder daß die Öffnung des Hohlraums (24) durch die Seitenwand der Kammer (17) dicht verschlossen wird (Fig. 6). Die Variante 4g zeigt eine andere Ausführungsform eines Kolbens (21) der in Fig. 4f dargestellten Art im axialen Schnitt, wobei der Hohlraum (24) durch eine Trennwand in zwei Kammern zur getrennten Aufbewahrung von verschiedenen Inhaltsstoffen der Kammer (17) aufgeteilt ist. Auch hier dichtet die Abtrennung (20) den Kolben an seiner offenen Seite hin ab. Die Öffnung des Kolbens (21) gemäß Fig. 4f und Fig. 4g kann sich entweder über die gesamte Breite erstrecken (dargestellt) oder aber nur über einen Teil davon (nicht dargestellt).

Um ein Verkanten des Kolbens (21) zu verhindern, können an dem Kolben und /oder der Seitenwandung der Kammer (17) auch Führungseinrichtungen ausgebildet sein, z.B. Führungsschienen oder Führungsrippen (nicht abgebildet).
Zur Verbesserung des Gleitens des Kolbens (21) durch die Öffnung (18) und die Kammer (17) kann der Kolben (21) oder die Wand der Kammer (17) mit einem pharmakologisch verträglichen Schmiermittel beschichtet sein. Derartige Schmiermittel sind dem Stand der Technik bekannt und umfassen z.B. Sorbitanester, z.B. Sorbitantrioleat, Ölsäure, Lecithin und andere Fettsäuren, Fettalkohole, Ester von Fettsäuren und dergleichen.

Die Führungsröhre (16) für Kanülen oder Kapillaren kann so ausgebildet sein, daß sie eine passgenaue Kapillare (15) dicht zum Stutzen (7) hin versiegelt, wenn sie in den Stutzen (7) hineingesteckt wird. Gegebenenfalls verjüngt sich die Führungsröhre (16) mit zunehmendem Abstand vom Kopf der Verschlußkappe (3). Alternativ oder ergänzend hierzu kann in der Führungsröhre (16) an einer beliebigen Stelle ein durchstechbares Septum bzw. eine elastischer O-Ring-Dichtung (23) angebracht sein. Der Einfachheit halber wird im weiteren Verlauf der Beschreibung der Ausdruck "Septum (23)" stellvertretend für beides, ein durchstechbares Septum und eine elastischer O-Ring-Dichtung (23) benutzt. Wie der Name bereits ausdrückt, ist es die Aufgabe der Führungsröhre (16) die Kanüle oder Kapillare (15) ohne Komplikationen auf einem vorgegebenen Weg durch die Verschlußkappe (3) zu führen.

An der zum Kopfende hin offenen Seite kann die Führungsröhre (16) einen Originalitätsverschluß oder einen Schutz vor Verunreinigungen aufweisen. Gegebenenfalls können diese Aufgaben auch von dem Septum (23) übernommen werden.

In einer bevorzugten Ausführungsform weist die Kammer (17) über die gesamte Längsachse einen gleichbleibenden Innendurchmesser auf. Die Öffnungen (18) und (19) befinden sich senkrecht zur Längsachse an der Oberseite bzw. der Unterseite der Kammer (17). Beide Öffnungen erstrecken sich über den gesamten Durchmesser der Kammer (17). Der Kolben (21) kann einen geringfügig größeren Außendurchmesser als der Innendurchmesser der Kammer (17) aufweisen, insbesondere wenn er sich in Verschlußstellung innerhalb der Kammer (17) befindet (Fig. 3). Dadurch wird ein besserer Verschluß der Öffnung (18) erreicht. Zusätzlich hat das den Vorteil, daß der Kolben (21) die Kammer (17) vollständig entleert, wenn er durch sie hindurch geschoben wird.

Wie bereits beschrieben, sind in den Figuren 5 und 6 Ausführungsformen mit einem hohlen Kolben (21) dargestellt, bei denen der (die) Inhaltsstoff(e) (22) in dem Hohlraum (24) des Kolbens gelagert wird (werden), anstelle direkt in der Kammer (17). In solchen Ausführungsformen schiebt die Kanüle (15) den Kolben (21) gänzlich durch die Kammer (17) bis der Kolben in den Behälter (2) fällt und der Inhalt (22) des Kolbens (21) durch das Lösungsmittel in dem Behälters (2) gelöst oder suspendiert wird.

Eine derartige Variante kann auch so ausgebildet sein, daß der Kolben nicht gänzlich durch die Kammer (17) geschoben werden kann, sondern nur soweit, daß sich der Inhalt (22) in den Behälter (2) ergießen kann (nicht dargestellt). In diesem Fall sind an der Außenwand des Kolbens (21) und/oder der Innenwand der Kammer (17) Führungsschienen und/oder Arretierungen angebracht, die verhindern, daß der Kolben ganz durch die Kammer (17) gedrückt wird (nicht dargestellt). In diesem Fall kann auch die Seitenwand der Kammer (17) eine Öffnung aufweisen, die von dem Kolben (21) so lange dicht verschlossen wird, bis der Kolben (21) durch die Kanüle (15) wegbewegt wird. Dabei ist die Öffnung so ausgebildet, daß dann durch sie die fertige Aerosolformulierung mittels der Kanüle (15) entnommen werden kann. Kolben (21), Kammer (17), die Kanüle (15) und die Führungsschienen und/oder Arretierungen sind dabei so ausgebildet, daß der Kolben (21) die Kanüle (15) nicht blockieren kann, nachdem er von ihr in seine Endposition geschoben wurde. Gegebenenfalls bewirkt die Kanüle (15) nur die Anfangsbewegung des Kolbens (21) durch die Kammer (17) und anschließend fällt der Kolben (21) z.B. aufgrund der Schwerkraft oder aufgrund eines weiteren Mechanismus in die Arretierung der Endposition. Dadurch wird verhindert, daß der Kolben (21) die Kanüle (15) blockiert.

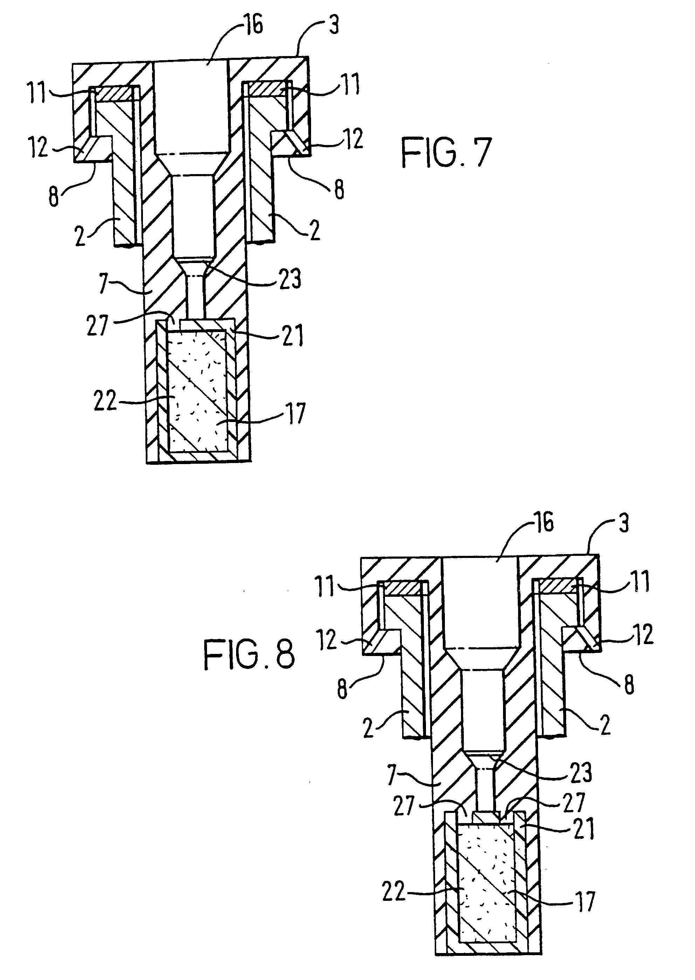

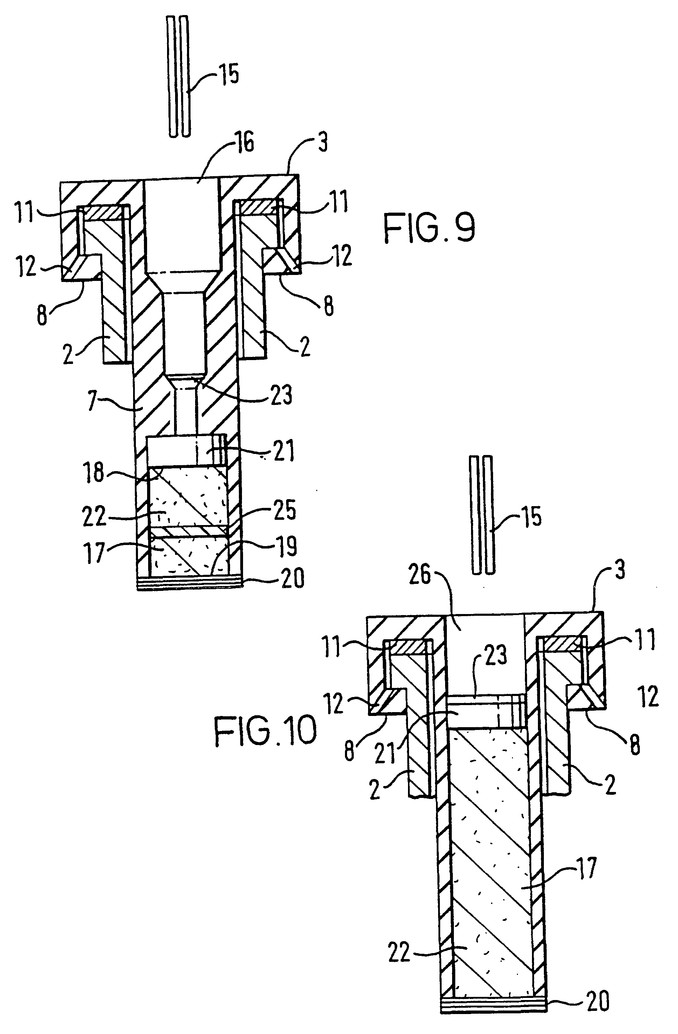

In Figur 7 ist eine weitere Variante mit einem hohlen Kolben (21) dargestellt, der die Kammer (17) gänzlich ausfüllt. Der Kolben (21) ist mit seiner Öffnung auf einen in dem Stutzen (7) ausgebildeten stopfenartigen Vorsprung (27) aufgesteckt, der gleichzeitig den Kolben (21) in der Kammer (17) hält und die Öffnung des Kolbens (21) dicht verschließt. In dieser Variante ist die Öffnung des Kolbens (21) so ausgebildet, daß nur ein Teil der Seitenwand offen ist. Die Führungsröhre (16) führt bis zu einem geschlossenen Teil der Seitenwand des Kolbens (21), so daß die Kanüle (15) an dieser Stelle ansetzen kann, um den Kolben (21) aus seiner Verankerung mit dem Vorsprung (27) zu lösen und in den Behälter (2) zu stoßen. Der Kolben (21) kann auch mehrere Öffnungen aufweisen, die durch mehrere stopfenartige Vorsprünge (27) in dem Stutzen (7) verschlossen sind (Fig. 8).

In solchen Varianten braucht die Innenöffnung (19) nicht notwendigerweise durch eine Abtrennung (20) verschlossen zu werden, doch könnte dies erfolgen (nicht dargestellt).

Fig. 9 zeigt eine Ausführungsform der Verschlußkappe (3) ähnlich der in Fig. 3 dargestellten, bei der die Kammer (17) durch eine Abtrennung (25) in zwei Kompartimente aufgeteilt ist. Die Abtrennung (25) ist ähnlich wie die Abtrennung (20) leicht zu öffnen oder entfernen. Die Kompartimente dienen zur getrennten Aufnahme von zwei oder mehr Wirkstoffen oder Zusatzstoffen. Analog können auch mehr als zwei Kompartimente ausgebildet sein (nicht dargestellt).

In einer Ausführungsform, bei der die Kammer (17) wenigstens zwei Kompartimente aufweist, kann in einem der Kompartimente, z.B. dem Kompartiment, das nicht durch die Abtrennung (20) verschlossen ist (oberes Kompartiment), ein pharmakologisch verträgliches Lösungsmittel mit sehr gutem Lösungsvermögen für den Wirkstoff gelagert werden. Ein solches Lösungsmittel kann z.B. von der oben definierten Art oder andere dem Stand der Technik bekannte Lösungsmittel sein, z.B. reiner Ethanol.

In einer solchen Ausführungsform kann der Kolben (21) mit einem oder mehreren Domen oder Schneiden versehen sein. In einem Zwei-Stufen-Mechanismus wird der Kolben (21) zunächst soweit in die Kammer gedrückt, daß nur die Abtrennung (25) geöffnet wird und das Lösungsmittel aus dem oberen Kompartiment in das untere Kompartiment läuft und den darin befindlichen Wirkstoff (22) auflöst oder suspendiert. In einem zweiten Schritt kann dann der Kolben (21) weiter bewegt werden, so daß die Abtrennung (20) geöffnet wird und sich der gelöste oder suspendierte Wirkstoff in den Behälter (2) ergießt.

Auch kann in diesem Fall anstelle des Kolbens (21) eine durchstechbare Membran ausgebildet sein. Die Dimension der Führungsröhre (16) und des oberen Kompartiments ist in diesem Fall derart, daß die Kanüle (15) gerade die Abtrennung (25) durchstechen oder aufreißen kann, so daß das Lösungsmittel in das untere Kompartiment läuft und den Wirkstoff auflöst oder suspendiert. Die Abtrennung (20) ist dann so beschaffen oder mit dem Stutzen verbunden, daß durch das Lösungsmittel die Verbindung der Abtrennung (20) mit dem Stutzen (7) aufgehoben wird, so daß sich die eben beschriebene Wirkstofflösung oder -suspension in den Behälter (2) vollständig ergießen kann.
Die zuletzt beschriebenen Varianten sind dann von Vorteil, wenn der Wirkstoff in der Kammer (17) ein Feststoff ist, der sich nicht ausreichend schnell in dem für die pharmazeutische Formulierung vorgesehenen Lösungsmittel auflöst oder suspendiert.

Alternativ kann das Durchmischen des Lösungsmittels in dem oberen Kompartiment mit dem (den) Inhaltsstoff(en) (22) im unteren Kompartiment auch unmittelbar vor dem Einsetzen der Kartusche in den Inhalator durchgeführt werden, z.B. indem der Benutzer die Verschlußkappe (3) gegen den Behälter (2) drückt und dadurch über einen diesbezüglich geeignete Mechanik die erste Stufe des Mechanismus durchführt. Ein derartiger Mechanismus kann unabhängig von der Anzahl der Kompartimente in der Kammer (17) ausgebildet sein, d.h. gegebenenfalls ist in der Kammer (17) nur ein einziges Kompartiment ausgebildet.

Fig. 10 zeigt eine Ausführungsform analog der Fig. 3, bei der an der Verschlußkappe (3) anstelle der passgenauen Führungsröhre (16) für die Kapillare (15) eine breitere Führungsröhre (26) ausgebildet ist. In dieser Ausführungsform wird der Kolben (21) auf der zum Kopf der Verschlußkappe (3) hin orientierten Seite durch ein durchstechbares Septum bzw. eine elastische O-Ring-Dichtung (23) gehalten. Auch in diesem Fall kann die Führungsröhre (26) durch einen Originalitätsverschluß oder einen Schutz vor Verunreinigung an ihrem offenen Ende am Kopf der Verschlußkappe verschlossen sein.

In anderen Ausführungsformen ist keine Führungsröhre (16) bzw. keine Röhre (26) ausgebildet. In diesen Fällen ist in der Oberseite der Verschlußkappe (3) ein Septum bzw. eine elastische O-Ring-Dichtung (23) integriert, unter dem sich unmittelbar der Kolben (21) oder die Kammer (17) befindet. Eine solche Ausführungsform ist zeichnerisch nicht dargestellt, ähnelt jedoch sehr stark der in Fig. 10 dargestellten Ausführungsform.

In den bisher beschriebenen Ausführungsform(en) der Figuren 3 bis 10 sind Kammer (17), Öffnung (18), Öffnung (19) und Kolben (21) so dimensioniert, daß der Kolben (21) die Öffnung (18) fest verschließt, aber durch Druck der Kapillare (15) vollständig durch die Kammer (17) und die Öffnung (19) hindurch geschoben werden kann. Im Fall der Ausführungsformen der Figuren 3 bis 6 und 9 und 10 öffnet sich die Abtrennung (20) durch die Bewegung des Kolbens (21) in die Kammer (17) aufgrund der durch die Kapillare bzw. Kanüle (15) ausgeübten Kraft, indem sie z.B. aufgrund der Druckzunahme im Inneren der Kammer (17) reißt und/oder durch einen oder mehrere am Kolben (21) ausgebildete(n) Dorn(e) oder Schneide(n) aufgestochen wird. Durch das Öffnen der Abtrennung (20) ergießt sich der Inhalt der Kammer (17) in den Behälter (2) mit dem Lösemittel. Gestalt und Anordnung der für diesen Vorgang beteiligten Elemente sind derart, daß die Kanüle (15) mit dem Inhalt der Kammer nicht in Berührung kommt, sowohl bevor als auch nachdem er mit dem Inhalt des Behälters (2) vermischt wird (wurde). Dadurch wird ein Verstopfen der Kanüle (15) verhindert.

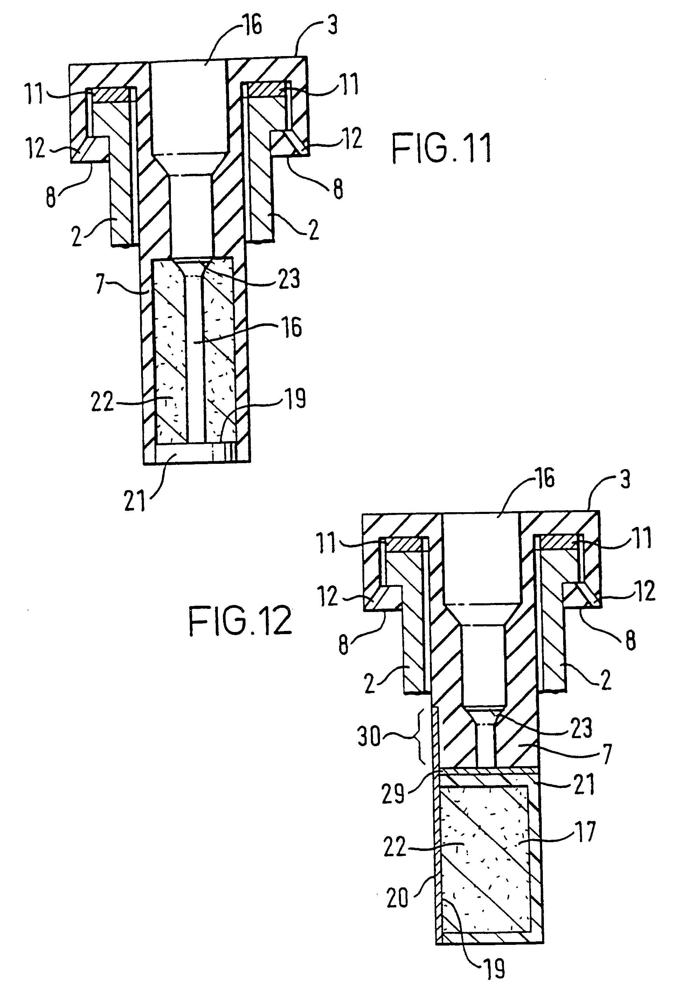

Eine andere Ausführungsform ist in Fig. 11 dargestellt und betrifft eine Verschlußkappe (3) bei der der Stutzen (7) innen weitgehend hohl ist und dadurch die Kammer (17) bildet. Die Führungröhre (16) führt vom Kopf der Verschlußkappe (3) durch die Kammer (17) bis zum Kolben (21), der von der unteren Seitenwand der Kammer (17) gehalten wird und die Kammer (17) und die Führungsröhre (16) dicht verschließt. Der Kolben (21) verschließt gleichzeitig die Führungsröhre (16) gegenüber der Kammer (17). In der Führungsröhre befindet sich ein Septum (23). Der Kolben (21) ist derart ausgebildet, daß er mittels der Kanüle oder Kapillare (15) in den Behälter (2) hineingedrückt werden kann. Gegebenenfalls kann die Kammer (17) durch Trennwände (nicht dargestellt), die parallel zur Längsachse des Stutzens ausgebildet sind, in verschieden Kompartimente geteilt werden, die alle durch den Kolben (21) versiegelt werden (nicht dargestellt).

In Fig. 12 ist eine Verschlußkappe mit einem Stutzen (7) dargestellt, in dem eine Führungsröhre (16) mit einem durchstechbaren Septum (23) ausgebildet ist. Die Führungsröhre (16) reicht bis zum Fuß des Stutzen (7), wo über Sollbruchstellen (29) die Kammer (17) aufgehängt ist. In dieser Ausführungsform liegt die Öffnung (19) parallel zur Längsachse des Stutzens (7) und ist mit der Abtrennung (20) dicht verschlossen. Die Abtrennung (20) reicht über die Öffnung (19) hinaus und ist an der Stelle (30) fest mit dem Stutzen (7) verbunden. Durchstößt eine Kapillare (15) das Septum (23) und drückt auf die Kammer (17), reißen zunächst die Sollbruchstellen (29) ab. Durch das Eigengewicht der Kammer (17) oder den weiteren Druck durch die Kanüle (15) wird dann die Abtrennung (20) über der Öffnung (19) so aufgerissen, daß die Abtrennung (20) weiterhin mit dem Stutzen (7) verbunden bleibt, die Kammer (17) selbst jedoch in den Behälter (2) fällt. Gegebenenfalls können die Sollbruchstellen (29) als eine leicht abreißbare Folie oder Klebestreifen ausgebildet sein, die (der) an der gegenüber der Abtrennung (20) liegenden Seite die Kammer (17) mit dem Stutzen (7) verbindet.
Alternativ kann die Kammer (17) ausschließlich über die Abtrennung (20) mit dem Stutzen (7) verbunden sein. In diesem Fall fehlen die Sollbruchstellen (29).

Falls ein Wirkstoff als Lösung appliziert werden soll und als Suspensionen in der Kammer (17) aufbewahrt wird, werden Ausführungsformen gemäß der Fig. 3, 4, 9 und 10 bevorzugt, insbesondere solche bei denen die Abtrennung (20) in Form einer Siegelfolie mit einer Aluminiumschicht ausgebildet ist. Diese Ausführungsformen haben den Vorteil, daß durch Druck des Kolbens (21) in der Kammer (17) ein Überdruck aufgebaut wird, der dazu führt, daß die Abtrennung (20) reißt und die Suspension mit Druck aus der Kammer (17) austritt und sich schnell in dem Lösungsmittel in dem Behälter (2) löst.

Behälter und Verschlußkappe werden im allgemeinen aus einem verformbaren Kunststoff gefertigt. Die Wandungen des Behälters sind so gefertigt, daß sie bei der Entnahme der Flüssigkeit ausreichend nachgeben bzw. zusammenfallen. Die Abtrennung (20) besteht im allgemeinen aus einer dünnen Kunststoffolie. Bevorzugt enthält die Abtrennung (20) eine dünne Aluminiumfolie.

Die erfindungsgemäße Kartusche mit Arzneimittelformulierungen für ein Inhalationsgerät soll eine lange Lagerfähigkeit aufweisen. Hierzu ist es erforderlich, daß das Lösungsmittel aus dem Behälter (2) vor Gebrauch nicht in die Kammer (17), die den Wirkstoff enthält, diffundieren kann. Neben einer ausreichenden Wandstärke der Kammer, kann zusätzlich eine Aluminiumbeschichtung auf den Außenflächen oder den Innenflächen der Kammer (17) aufgebracht sein.

Die Kartusche ist bevorzugt derart dimensioniert sein, daß sie in Inhalatoren, wie den eingangs offenbarten, eingesetzt werden kann. Das bevorzugte Fassungsvermögen des Behälters (2) beträgt ca. 5 ml, das der Kammer (17) von 0,001 ml bis zu 0,5 ml, bevorzugt von 0,001 ml bis zu 0,2 ml, besonders bevorzugt von 0,001 ml bis zu 0,05 ml, ganz besonders bevorzugt von 0,001 ml bis zu 0,03 ml.

Für die Herstellung solcher Behälter wie auch für die Verschlußkappe stehen dem Fachmann geeignete Kunststoffe - beispielsweise aus Polystyrol (PS), Polycarbonat (PC), Polymethylmethacrylat (PMMA), Acrylnitril-Butadien-Styrol-Coploymere (ABS), Polyethylenterephthalat (PET) und andere zur Verfügung.

Es ist hervorzuheben, daß das Einsetzen der Kartusche mit der Kammer (17) in den Inhalator für den Patienten im wesentlichen die gleichen Handgriffe erfordert wie das Einsetzen einer gewöhnlichen Kartusche.

### Beispiele für das erfindungsgemäße Wirkstoffkonzentrat

Im folgenden werden beispielhaft einzelne Formulierungen mit Konzentrations- oder Mengenangaben aufgeführt. Die Beispiele stellen jedoch nur eine Auswahl aus den oben genannten möglichen Formulierungskombinationen dar.

### Beispiel 1

5 mg Formoterol (Teilchengröße: 5 µm) werden als Suspension mit 0,015 ml Wasser in der Kammer (17) dosiert. Durch Fumarsäure wird ein pH-Wert von 5,0 eingestellt. Die Konzentration des Formoterol beträgt 333 mg/ml.
Im Behälter (2) werden 4,5 ml einer 1:1 Lösung Wasser/Ethanol (v/v) vorgelegt. Die Lösung enthält 0,45 mg Benzalkoniumchlorid und 2,25 mg Na- EDTA und ist mit HCI auf einen pH-Wert von 5,0 eingestellt.

Nach Vermischen beträgt die Formoterol-Konzentration der zu applizierenden Formulierung ca. 1,1 mg/ml.

### Beispiel 2

5 mg Formoterol (Teilchengröße: 5 µm) werden Suspension mit 0,015 ml einer 20 Gew% wäßrigen NaCl-Lösung in der Kammer (17) dosiert. Durch HCI wird ein pH-Wert von 5,0 eingestellt.
Im Behälter (2) werden 4,5 ml einer 1:1 Lösung Wasser/Ethanol (v/v) vorgelegt. Die Lösung enthält 0,45 mg Benzalkoniumchlorid und 2,25 mg Na- EDTA und ist mit HCI auf einen pH-Wert von 5,0 eingestellt.

Analog werden Kartuschen mit Formoterol als Wirkstoff bereit gestellt, bei denen der Gehalt an Formoterol bevorzugt zwischen 100 und 400 mg/ml, besonders bevorzugt zwischen 250 und 350 mg/ml liegt.

### Beispiel 3

In dem Behälter (2) werden in 4,5 ml Wasser als Lösungsmittel 0,1 Gew.% Tiotropiumbromid, 0,01 Gew.% Benzalkoniumchlorid, 0,05 Gew.% EDTA formuliert. Mit Salzsäure wird ein pH-Wert von 3,0 eingestellt.
In der Kammer (17) befindet sich eine 10 mg schwere Tablette bestehend aus 0,5 mg der Puffersubstanz Na₂HPO₄ x 2H₂O und 9,5 mg NaCI.
Beim Einsatz der Kartusche in den Inhalator wird die Puffersubstanz der Kammer (17) mit der Lösung in dem Behälter (2) vermischt und dadurch ein pH-Wert von 3,5 eingestellt.

Analog werden Kartuschen mit Tiotropiumbromid als Wirkstoff bereit gestellt, bei denen der Gehalt an Tiotropiumbromid bevorzugt zwischen 0,002 und 0,4 Gew.% liegt, besonders bevorzugt zwischen 0,0005 und 0,2 Gew. Der pH-Wert der Lösung im Behälter (2), vor Vermischung mit dem Puffer in der Kammer (17) liegt in diesen Fällen bevorzugt unter 4,0, besonders bevorzugt zwischen 2,0 und 3,0 und ganz besonders bevorzugt zwischen 2,5 und 3,0.

## Patentansprüche

1. Vorrichtung aus Verschlußkappe (3) und einem Behälter (2) in Form einer Kartusche zur getrennten Lagerung eines Wirkstoffs und einer pharmakologisch verträglichen Flüssigkeit, wobei die Verschlußkappe (3) ein Stutzen (7) aufweist, der während des Aufschiebens der Verschlußkappe (3) auf den Hals des Behälters (2) in den Behälter eintaucht und einen Teil des Behälterinhalts verdrängt, durch die Verschlußkappe (3) führt eine Führungsröhre (16) zum Durchführen einer Kapillare oder Kanüle (15), die bis zu einer Kammer (17) reicht, die fest mit dem Stutzen (7) verbunden ist oder darin integriert ist und mindestens eine Öffnung enthält, **dadurch gekennzeichnet, daß** die Kammer (17) durch einen beweglichen Kolben (21) fest verschlossen ist und die Führungsröhre (16) bis zu dem Kolben (21) führt.

2. Vorrichtung nach Anspruch 1 **dadurch gekennzeichnet, daß** sich in der Verschlußkappe zwischen Kolben (21) und Außenumgebung ein durchstoßbares Septum (23) befindet.

3. Vorrichtung nach einem der vorangegangenen Ansprüche 1 bis 2, **dadurch gekennzeichnet, daß** in der Führungsröhre (16) ein durchstoßbares Septum bzw. eine elastische O-Ring-Dichtung (23) ausgebildet ist.

4. Vorrichtung nach einem der vorangegangen Ansprüche 1 bis 3, **dadurch gekennzeichnet, daß** die Kammer eine weitere Öffnung enthält, die durch eine durchstoßbare, abreißbare oder bewegliche Abtrennung (20) dicht verschlossen ist.

5. Vorrichtung nach Anspruch 4, **dadurch gekennzeichnet, daß** die Abtrennung (20) als eine Siegelfolie ausgebildet ist.

6. Verschlußkappe nach einem der vorhergehenden Ansprüche1 bis 5, **dadurch gekennzeichnet, daß** die Kammer (17) zwei oder mehr Kompartimente enthält, die übereinander angeordnet sind und jeweils durch eine durchstoßbare, aufreißbare oder bewegliche Abtrennung (25) gegeneinander dicht abgeschlossen sind.

7. Verschlußkappe, nach einem der vorangegangen Ansprüche 1 bis 6, **dadurch gekennzeichnet, daß** der Kolben (21) die Form eines Zylinders mit einem Verhältnis Länge zu Durchmesser von 1,2 bis 2,0 aufweist.

8. Verschlußkappe nach einem der vorhergehenden Ansprüche 1 bis 7, **dadurch gekennzeichnet, daß** die zur Kammer (17) hin orientierte Seite des Kolbens (21) abgeschrägt ist, spitz zuläuft und/ oder an der Seite, die zur Kammer (17) hin orientiert ist, einen oder mehrere Dom(e) oder eine oder mehrere Schneide(n) hat.

9. Vorrichtung nach einem der vorangegangen Ansprüche 1 bis 8, **dadurch gekennzeichnet, daß** die Kammer (17) mit einem passgenauen Kolben (21) ausgefüllt ist, der einen Hohlraum (24) beinhaltet, der an einer Seite eine Öffnung aufweist.

10. Vorrichtung nach einem der vorangegangen Ansprüche 1 bis 9, **dadurch gekennzeichnet, daß** die Öffnung des Hohlraums (24) durch die Abtrennung (20) oder die Seitenwand der Kammer (17) versiegelt ist.

11. Vorrichtung nach Anspruch 10 **dadurch gekennzeichnet, daß** der Hohlraum (24) durch Trennwände in zwei oder mehrere voneinander dicht abgetrennte Kompartimente aufgeteilt ist und daß jedes Kompartiment durch die Abtrennung (20) oder die Seitenwand der Kammer (17) versiegelt ist.

12. Vorrichtung nach einem der vorangegangen Ansprüche 1 bis 11, **dadurch gekennzeichnet, daß** in dem Kolben (21) eine Aussparung (28) ausgebildet ist, die mit ihrer Öffnung zum Kopf der Verschlußkappe (3) hin orientiert ist.

13. Vorrichtung nach einem der vorangegangen Ansprüche 1 bis 12, **dadurch gekennzeichnet, daß** der Kolben (21) und/oder die Wand der Kammer (17) mit einem pharmakologisch verträglichen Schmiermittel, bevorzugt Ölsäure, Lecithin oder ein Sorbitanester, wie z.B. Sorbitantrioleat, beschichtet ist.

14. Vorrichtung nach einem der vorangegangen Ansprüche 1, 2, 3, 7, 8, 9, 10, 11, 12 oder 13, **dadurch gekennzeichnet, daß** die Kammer (17) nur eine Öffnung (19) am unteren Ende des Stutzens (7) enthält, die durch einen Kolben (21) dicht verschlossen ist und daß die Führungsröhre (16) für Kapillaren oder Kanülen durch die Kammer (17) bis zum Kolben (21) führt.

15. Vorrichtung nach Anspruch 14, **dadurch gekennzeichnet, daß** die Kammer so in mehrere Kompartimente aufgeteilt ist, daß alle Kompartimente durch den Kolben (21) versiegelt werden.

16. Vorrichtung nach einem der vorangegangen Ansprüche 9, 10, 11, 12, 13, 14 oder 15, **dadurch gekennzeichnet, daß** der Kolben (21) mit dem Hohlraum (24) über eine oder mehrere Öffnungen des Kolbens (21) durch einen oder mehrere an dem Stutzen (7) ausgebildete(n) stopfenartige(n) Vorsprünge (Vorsprung) (27) befestigt ist.

17. Vorrichtung nach einem der vorangegangen Ansprüche 1 bis 16, **dadurch gekennzeichnet, daß** der Kolben (21) bis zu 5 % breiter ist, als die Öffnung, die er verschließt.

18. Vorrichtung nach einem der vorangegangen Ansprüche 1 bis 17, **dadurch gekennzeichnet, daß** die Kammer (17) eine Arzneimittelkonzentrat, bevorzugt eine Suspension oder Lösung, insbesondere bevorzugt eine Suspension, in einer pharmakologisch verträglichen treibmittelfreien Flüssigkeit, bevorzugt Wasser, eine wäßrige Lösung mit einem pharmakologisch verträglichen Salz, Ethanol oder ein Gemisch davon, mit einem oder mehreren Wirkstoffe(n) enthält, wobei der oder die Wirkstoff(e) insbesondere für die inhalative Therapie geeignet (ist) sind.

19. Vorrichtung nach Anspruch 18, **dadurch gekennzeichnet, daß** der oder die Wirkstoff(e) aus der Gruppe der Betamimetica, Anticholinergika, Antiallergika, Leukotrien-Antagonisten und / oder Steroide ausgewählt ist (sind).

20. Vorrichtung nach Anspruch 18 oder 19, **dadurch gekennzeichnet, daß** der oder die Wirkstoffe) Formoterol, Tiotropium und/oder Salbutamol und/oder ein Salz davon (ist) sind.

21. Vorrichtung nach einem der vorangegangen Ansprüche 18 bis 20, **dadurch gekennzeichnet, daß** die Konzentration der Wirkstoffe zwischen 75 mg/ml und 1000 mg/ml liegt, insbesondere bevorzugt zwischen 75 mg/ml und 500 mg/ml, ganz besonders bevorzugt zwischen 100 mg/ml und 400 mg/ml.

22. Vorrichtung nach Anspruch 20 oder 21, **dadurch gekennzeichnet, daß** die Kammer (17) eine Suspension von Formoterol in einer Konzentration zwischen 75 mg/ml und 500 mg/ml, bevorzugt zwischen 100 und 400 mg/ml, besonders bevorzugt zwischen 250 und 350 mg/ml enthält.

23. Vorrichtung nach einem der Ansprüche 1 bis 22, **dadurch gekennzeichnet, daß** das Wirkstoffkonzentrat in der Kammer (17) eine Säure, ein Konservierungsmittel und / oder einen Komplexbildner enthält

24. Vorrichtung nach einem der vorangegangen Ansprüche 1 bis 23, **dadurch gekennzeichnet, daß** der Behälter (2) eine pharmakologisch verträgliche, reibmittelfreie Flüssigkeit, bevorzugt Wasser, eine wäßrige Salzlösung, Ethanol oder ein Gemisch davon, enthält, die gegebenenfalls eine Säure, einen Komplexbildner, ein Konservierungsmittel, ein oberflächenaktives Mittel und/oder eine Puffersubstanz enthält

25. Vorrichtung nach Anspruch 24, **dadurch gekennzeichnet, daß** das Lösungsoder Suspensionsmittel einen oder mehrere Arzneistoff(e) enthält, die bevorzugt aus der Gruppe der Betamimetica, Anticholinergika, Antiallergika, Leukotrien-Antagonisten und / oder Steroide ausgewählt ist (sind).

26. Vorrichtung nach einem der Ansprüche 24 oder 25, **dadurch gekennzeichnet, daß** die Kammer (17) eine Puffersubstanz, bevorzugt Nartrium- oder Kaliumdihydrognephosphat und/oder Dinatrium- oder Dikaliumhydrogenphosphat und/oder Gemische daraus beinhaltet

27. Vorrichtung nach einem der Ansprüche 18 bis 26, **dadurch gekennzeichnet, daß** der Behälter eine Lösung von Tiotropiumbromid mit einem Anteil zwischen 0,002 und 0,4 Gew.%, bevorzugt zwischen 0,0005 und 0,2 Gew.%, besonders bevorzugt von 0,1 Gew.% enthält, die eine pH-Wert von 2,0 bis 4,0, bevorzugt 2,0 bis 3,0 und besonders bevorzugt 2,8 aufweist.

28. Vorrichtung nach einem der vorangegangen Ansprüche 1 bis 27, **dadurch gekennzeichnet, daß** die Verschlußkappe (3) zusammen mit einem Behälter (2) eine Kartusche für einen Zerstäuber bildet.

29. Verwendung einer Vorrichtung nach einem der Ansprüche 1 bis 28 als Container für flüssige Arzneimittelformulierungen in Inhalatoren zur Erzeugung von zur medizinischen Therapie, insbesondere für die inhalative Therapie geeigneten Aerosolen.

## Claims

1. An apparatus comprising a closure-cap (3) and a container (2) in the form of a cartridge for separate storage of an active ingredient and a pharmacologically acceptable liquid, wherein the closure-cap (3) has a connecting piece (7) which dips into the container and displaces some of the container contents when the closure-cap (3) is pushed onto the neck of the container (2), a guide tube (16) for guiding a capillary or canula (15) passes through the closure-cap (3) and extends as far as a chamber (17) which is firmly connected to the connecting piece (7) or integrated therein and contains at least one opening, **characterised in that** the chamber (17) is sealed tightly by amovable plunger (21) and the guide tube (16) leads up to the plunger (21).

2. An apparatus according to claim 1, **characterised in that** a penetrable septum (23) is located in the closure-cap between the plunger (21) and the outside environment.

3. An apparatus according to one of the preceding claims 1 and 2, **characterised in that** a penetrable septum or an elastic O-ring seal (23) is formed in the guide tube (16).

4. An apparatus according to one of the preceding claims 1 to 3, **characterised in that** the chamber has a further opening which is sealed tightly by a penetrable, tearable or movable separating element (20).

5. An apparatus according to claim 4, **characterised in that** the separating element (20) is formed as a sealing foil.

6. A closure-cap according to one of the preceding claims 1 to 5, **characterised in that** the chamber (17) has two or more compartments which are arranged one above the other and are each sealed from the other by a penetrable, tearable or movable separating element (25).

7. A closure-cap according to one of the preceding claims 1 to 6, **characterised in that** the plunger (21) has the shape of a cylinder with a ratio of length to diameter of 1.2 to 2.0.

8. A closure-cap according to one of the preceding claims 1 to 7, **characterised in that** the side of the plunger (21) facing the chamber (17) is sloped or tapers to a point and/or has one or more spike(s) or cutting edge(s) on the side which is orientated towards the chamber (17).

9. An apparatus according to one of the preceding claims 1 to 8, **characterised in that** the chamber (17) is filled with an exactly-fitting plunger (21) which contains a hollow space (24) which has an opening on one side.

10. An apparatus according to one of the preceding claims 1 to 9, **characterised in that** the opening of the hollow space (24) is sealed by the separating element (20) or by the side wall of the chamber (17).

11. An apparatus according to claim 10, **characterised in that** the hollow space (24) is divided by partition walls into two or more compartments which are tightly sealed from one another and that each compartment is sealed by the separating element (20) or by the side wall of the chamber (17).

12. An apparatus according to one of the preceding claims 1 to 1.1, **characterised in that** a recess (28) is formed in the plunger (21) which is orientated with its opening towards the head of the closure-cap (3).

13. An apparatus according to one of the preceding claims 1 to 12, **characterised in that** the plunger (21) and/or the wall of the chamber (17) is coated with a pharmacologically acceptable lubricant, preferably oleic acid, lecithin or a sorbitan ester such as sorbitan trioleate,

14. An apparatus according to one of the preceding claims 1, 2, 3, 7, 8, 9, 10, 11, 12 or 13, **characterised in that** the chamber (17) has only one opening (19) at the lower end of the connecting piece (7) which is sealed tightly by a plunger (21), and that the guide tube (16) for capillaries or canulae leads through the chamber (17) to the plunger (21).

15. An apparatus according to claim 14, **characterised in that** the chamber is divided into a plurality of compartments in such a way that all the compartments are sealed by the plunger (21).

16. An apparatus according to one of the preceding claims 9, 10, 11, 12, 13, 14 or 15, **characterised in that** the plunger (21) with the hollow space (24) is attached via one or more openings of the plunger (21) by means of one or more stopper-like projection(s) (27) formed on the connecting piece (7).

17. An apparatus, according to one of the preceding claims 1 to 16, **characterised in that** the plunger (21) is up to 5% wider than the opening which it closes.

18. An apparatus according to one of the preceding claims 1 to 17, **characterised in that** the chamber (17) contains a pharmaceutical concentrate, preferably a suspension or solution, most preferably a suspension, in a pharmacologically acceptable propellant-free liquid, preferably water, an aqueous solution with a pharmacologically acceptable salt, ethanol or a mixture thereof, with one or more active substances, the active substance or substances being suitable in particular for inhalative therapy.

19. An apparatus according to claim 18, **characterised in that** the active substance (or substances) is (or are) selected from among the betamimetics, anticholinergics, antiallergics, leukotriene antagonists and/or steroids.

20. An apparatus according to claim 18 or 19, **characterised in that** the active substance (or substances) is (or are) formoterol, tiotropium and/or salbutamol and/or a salt thereof.

21. An apparatus according to one of the preceding claims 18 to 20, **characterised in that** the concentration of the active substances is between 75 mg/ml and 1000 mg/ml, more preferably between 75 mg/ml and 500 mg/ml, most preferably between 100 mg/ml and 400 mg/ml.

22. An apparatus according to claim 20 or 21, **characterised in that** the chamber (17) contains a suspension of formoterol in a concentration of between 75 mg/ml and 500 mg/ml, preferably between 100 and 400 mg/ml, most preferably between 250 and 350 mg/ml.

23. An apparatus according to one of claims 1 to 22, **characterised in that** the active substance concentrate in the chamber (17) contains an acid, a preservative and/or a complexing agent.

24. An apparatus according to one of the preceding claims 1 to 23, **characterised in that** the container (2) contains a pharmacologically acceptable propellant-free liquid, preferably water, an aqueous saline solution, ethanol or a mixture thereof, which optionally contains an acid, a complexing agent, a preservative, a surfactant and/or a buffer.

25. An apparatus according to claim 24, **characterised in that** the solvent or suspension agent contains one or more medicament(s) preferably selected from among the betamimetics, anticholinergics, antiallergics, leukotriene antagonists and/or steroids.

26. An apparatus according to one of claims 24 or 25, **characterised in that** the chamber (17) contains a buffer substance, preferably sodium or potassium dihydrogen phosphate and/or disodium or dipotassium hydrogen phosphate and/or mixtures thereof.

27. An apparatus according to one of claims 18 to 26, **characterised in that** the container contains a solution of tiotropium bromide in an amount of between 0.002 and 0.4 % by weight, preferably between 0.0005 and 0.2 % by weight, most preferably 0.1 % by weight, and having a pH of from 2.0 to 4.0, preferably 2.0 to 3.0 and most preferably 2.8.

28. An apparatus according to one of the preceding claims 1 to 27, **characterised in that** the closure-cap (3), together with a container (2), forms a cartridge for an atomiser.

29. Use of an apparatus according to one of the claims 1 to 28 as a container for liquid medicament formulations in inhalers for the production of aerosols suitable for medical treatment, especially for inhalative therapy.

## Revendications

1. Dispositif composé d'un capuchon (3) et d'un récipient (2) sous forme d'une cartouche pour stocker séparément un agent actif et un liquide pharmacologiquement compatible, le capuchon (3) présentant un ajoutoir (7) qui, lorsqu'on place le capuchon (3) sur le col du récipient (2), plonge dans le récipient et repousse une partie du contenu du récipient, un tube de guidage (16) conduisant à travers la capuchon (3) pour faire traverser un capillaire ou une canule (15) qui s'étend jusqu'à une chambre (17) solidaire de l'ajoutoir (7) ou intégrée à celui-ci et contenant au moins une ouverture, **caractérisé en ce que** la chambre (17) est fermée de façon étanche par un piston (21) mobile et le tube de guidage (16) conduit jusqu'au piston (21).

2. Dispositif selon la revendication 1, **caractérisé en ce qu'**un septum (23) transperçable se trouve dans le capuchon entre le piston (21) et le milieu extérieur.

3. Dispositif selon l'une des revendications précédentes 1 à 2, **caractérisé en ce qu'**un septum transperçable ou un anneau torique d'étanchéité (23) est formé dans le tube de guidage (16).

4. Dispositif selon l'une des revendications précédentes 1 à 3, **caractérisé en ce que** la chambre contient une autre ouverture qui est fermée hermétiquement par une séparation (20) transperçable, arrachable ou mobile.

5. Dispositif selon la revendication 4, **caractérisé en ce que** la séparation (20) est formée en tant que feuille de scellement.

6. Capuchon selon l'une des revendications précédentes 1 à 5, **caractérisé en ce que** la chambre (17) contient deux compartiments ou plus qui sont disposés l'un au-dessus de l'autre et sont à chaque fois hermétiquement isolés l'un de l'autre par une séparation (25) transperçable, arrachable ou mobile.

7. Capuchon selon l'une des revendications précédentes 1 à 6, **caractérisé en ce que** le piston (21) présente la forme d'un cylindre avec un rapport de la longueur au diamètre de 1,2 à 2,0.

8. Capuchon selon l'une des revendications précédentes 1 à 7, **caractérisé en ce que** le côté du piston (21) orienté vers la chambre (17) est biseauté, est effilé et/ou, sur le côté qui est orienté vers la chambre (17), a un ou plusieurs mandrin(s) ou une ou plusieurs lame(s).

9. Dispositif selon l'une des revendications précédentes 1 à 8, **caractérisé en ce que** la chambre (17) est remplie par un piston (21) exactement ajusté qui contient un espace creux (24) présentant une ouverture sur un côté.

10. Dispositif selon l'une des revendications précédentes 1 à 9, **caractérisé en ce que** l'ouverture de l'espace creux (24) est scellée par la séparation (20) ou par la paroi latérale de la chambre (17).

11. Dispositif selon la revendication 10, **caractérisé en ce que** l'espace creux (24) est divisé par des parois de séparation en deux compartiments ou plus qui sont isolés hermétiquement l'un de l'autre et **en ce que** chaque compartiment est scellé par la séparation (20) ou par la paroi latérale de la chambre (17).

12. Dispositif selon l'une des revendications précédentes 1 à 11, **caractérisé en ce que** dans le piston (21) est formé un évidement (28) qui est orienté avec son ouverture tournée vers la tête du capuchon (3).

13. Dispositif selon l'une des revendications précédentes 1 à 12, **caractérisé en ce que** le piston (21) et/ou la paroi de la chambre (17) est revêtu d'un lubrifiant pharmacologiquement compatible, de préférence de l'acide oléique, de la lécithine ou un ester de sorbitan comme par exemple du trioléate de sorbitan.

14. Dispositif selon l'une des revendications précédentes 1, 2, 3, 7, 8, 9, 10, 11, 12 ou 13, **caractérisé en ce que** la chambre (17) ne contient à l'extrémité inférieure de l'ajoutoir (7) qu'une ouverture (19) qui est fermée hermétiquement par un piston (21) et **en ce que** le tube de guidage (16) pour des capillaires ou pour des canules conduit à travers la chambre (17) jusqu'au piston (21).

15. Dispositif selon la revendication 14, **caractérisé en ce que** la chambre est divisée en plusieurs compartiments de telle manière que tous les compartiments sont scellés par le piston (21).

16. Dispositif selon l'une des revendications précédentes 9, 10, 11, 12, 13, 14 ou 15, **caractérisé en ce que** le piston (21) avec l'espace creux (24) est fixé au moyen d'une ou de plusieurs ouvertures du piston (21) par une ou plusieurs saillie(s) (27) formée(s) à la façon d'un tampon sur l'ajoutoir (7).

17. Dispositif selon l'une des revendications précédentes 1 à 16, **caractérisé en ce que** le piston (21) est jusqu'à 5% plus large que l'ouverture qu'il ferme.

18. Dispositif selon l'une des revendications précédentes 1 à 17, **caractérisé en ce que** la chambre (17) contient, en même temps qu'un ou plusieurs agent(s) actif(s), un concentré médicamenteux, de préférence une suspension ou une solution, plus particulièrement de préférence une suspension, dans un liquide pharmacologiquement compatible exempt d'agent moussant, de préférence l'eau, une solution aqueuse avec un sel pharmacologiquement compatible, de l'éthanol ou un mélange de ceux-ci, le ou les agent(s) actif(s) étant notamment adapté(s) pour un traitement par inhalation.

19. Dispositif selon la revendication 18, **caractérisé en ce que** le ou les agent(s) actif(s) est (sont) choisi(s) parmi le groupe des bêtamimétiques, des anticholinergiques, des antiallergiques, des antagonistes du leucotriène et/ou des stéroïdes.

20. Dispositif selon la revendication 18. ou 19, **caractérisé en ce que** le ou les agent(s) actif(s) est (sont) le formotémol, le tiotropium et/ou le salbutamol et/ou un sel de ceux-ci.

21. Dispositif selon l'une des revendications précédentes 18 à 20, **caractérisé en ce que** la concentration des agents actifs se situe entre 75 mg/ml et 1 000 mg/ml, particulièrement de préférence entre 75 mg/ml et 500 mg/ml, encore plus particulièrement entre 100 mg/ml et 400 mg/ml.

22. Dispositif selon la revendication 20 ou 21, **caractérisé en ce que** la chambre (17) contient une suspension de formotérol dans une concentration entre 75 mg/ml et 500 mg/ml, de préférence entre 100 mg/ml et 400 mg/ml, encore plus particulièrement entre 250 mg/ml et 350 mg/ml.

23. Dispositif selon l'une des revendications 1 à 22, **caractérisé en ce que** le concentré d'agent actif dans la chambre (17) contient un acide, un agent de conservation et/ou un agent complexant.

24. Dispositif selon l'une des revendications précédentes 1 à 23, **caractérisé en ce que** le récipient (2) contient un liquide pharmacologiquement compatible exempt d'agent moussant, de préférence de l'eau, une solution saline aqueuse, de l'éthanol ou un mélange de ceux-ci, qui éventuellement contient un acide, un agent complexant, un agent de conservation, un agent tensioactif et/ou un tampon.

25. Dispositif selon la revendication 24, **caractérisé en ce que** le solvant ou l'agent de suspension contient une ou plusieurs substance(s) médicamenteuse(s) qui est (sont) de préférence choisie(s) parmi le groupe des bêtamimétiques, des anticholinergiques, des antiallergiques, des antagonistes du leucotriène et/ou des stéroïdes.

26. Dispositif selon l'une des revendications 24 ou.25, **caractérisé en ce que** la chambre (17) contient un tampon, de préférence un hydrogénophosphate de sodium ou de potassium et/ou un hydrogénophosphate disodique ou dipotassique et/ou un mélange de ceux-ci.

27. Dispositif selon l'une des revendications 18 à 26, **caractérisé en ce que** le récipient contient une solution de bromure de tiotropium selon une quantité comprise entre 0,002 et 0,4 % en masse, de préférence entre 0,0005 et 0,2 % en masse, plus particulièrement de 0,1 % en masse, qui présente une valeur de pH entre 2,0 et 4,0, de préférence entre 2,0 et 3,0, plus particulièrement de 2,8.

28. Dispositif selon l'une des revendications précédentes 1 à 27, **caractérisé en ce que** le capuchon (3) forme avec un récipient (2) une cartouche pour un atomiseur.

29. Utilisation d'un dispositif selon l'une des revendications 1 à 28 en tant que conteneur pour des formulations liquides de médicament dans des inhalateurs pour produire des aérosols adaptés au traitement médical, en particulier pour des traitements par inhalation.
